(19) Europäisches Patentamt
European Patent Office
Office européen des brevets

(11) Numéro de publication: **0 077 721**
**B1**

# (12) FASCICULE DE BREVET EUROPEEN

(45) Date de publication du fascicule du brevet: **03.07.85**

(21) Numéro de dépôt: **82401871.7**

(22) Date de dépôt: **12.10.82**

(51) Int. Cl.⁴: **C 07 C 153/11**, C 07 D 233/72, A 01 N 53/00, A 23 K 1/16, A 61 K 31/265, A 61 K 31/275

(54) **Nouveaux dérivés de l'acide cyclopropane carboxylique comportant un groupement alcoylthio carbonyle, leur préparation, leur application à la lutte contre les parasites des végétaux, des animaux et des locaux et les compositions les renfermant.**

(30) Priorité: **16.10.81 FR 8119481**

(43) Date de publication de la demande: **27.04.83 Bulletin 83/17**

(45) Mention de la délivrance du brevet: **03.07.85 Bulletin 85/27**

(84) Etats contractants désignés: **CH DE FR GB IT LI NL**

(56) Documents cités: **FR - A - 2 185 612**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Tessier, Jean, 30, rue Jean Moulin, F-94300 Vincennes (FR)**
Inventeur: **Teche, André, 15, rue Godot de Mauroy, F-75009 Paris (FR)**

(74) Mandataire: **Tonnellier, Marie-José et al, ROUSSEL-UCLAF 111, route de Noisy Boîte Postale no.9, F-93230 Romainville (FR)**

**EP 0 077 721 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'acide cyclopropane carboxylique comportant un groupement alcoylthio carbonyle, leur préparation, leur application à la lutte contre les parasites des végétaux, des animaux et des locaux et les compositions les renfermant.

On connaissait déjà des dérivés de l'acide cyclopropane carboxylique portant en position 3 une chaîne latérale

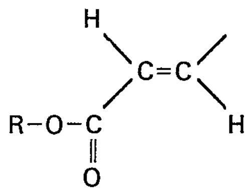

(voir à ce sujet le brevet français 2.185.612 ainsi que J. Chem. Soc. Perkin I page 2470, 1974). Toutefois, la géométrie de la chaîne latérale de ces composés est E de façon prédominante. En effet, le procédé de synthèse utilisé pour préparer ces dérivés ne fournissait pas exclusivement que la géométrie E (voir Agr. Biol. Chem. *34*, page 1119, 1970).

L'invention a pour objet sous toutes leurs formes stéréo-isomères ou sous forme de mélanges, les composés de formule (I):

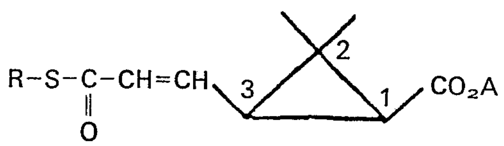 (I)

dans laquelle la copule cyclopropanique a la structure 1R cis et la double liaison a la géométrie Z, dans laquelle A représente

— soit un radical alcoyle renfermant de 1 à 18 atomes de carbone,

— soit un radical benzyle éventuellement substitué sur le cycle aromatique par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

— soit un groupement:

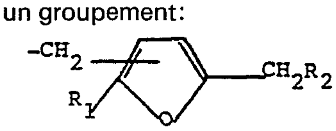

dans lequel le substituant $R_1$ représente un atome d'hydrogène ou un radical méthyle et le substituant $R_2$ un aryle monocyclique ou un groupement $-CH_2-C{\equiv}CH$ et notamment un groupement 5-benzyl 3-furyl méthyle,

— soit un groupement:

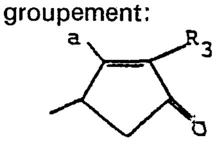

dans lequel a représente un atome d'hydrogène ou un radical méthyle et $R_3$ représente un radical organique aliphatique comportant de 2 à 6 atomes de carbone et une ou plusieurs insaturations carbone-carbone et notamment l'un des radicaux: $-CH_2-CH{=}CH_2$, $-CH_2-CH{=}CH-CH_3$, $-CH_2-CH{=}CH-CH{=}CH_2$, ou $-CH_2-CH{=}CH-CH_2-CH_3$,

— soit un groupement:

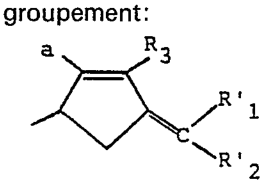

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone ou un groupement cyano,

— soit un groupement:

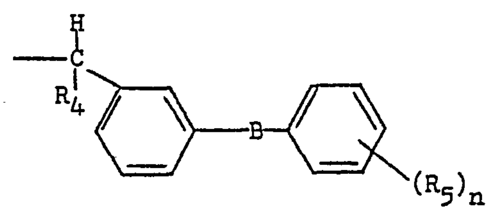

dans lequel B représente un atome d'oxygène ou de soufre ou un groupement:

O<br>‖<br>$-C-$ ou $-CH_2-$ et $R_4$ représente un atome d'hydrogène, un radical $-C{\equiv}N$, un radical méthyle, un radical $-COHN_2$, un radical $-CSNH_2$ ou un radical $-C{\equiv}CH$, $R_5$ représente un atome d'hylogène ou un radical méthyle et n représente un nombre égal à 0, 1 ou 2, et notamment le groupement 3-phénoxy benzyle, α-cyano 3-phénoxy benzyle, α-éthynyl 3-phénoxy benzyle, 3-benzoyl benzyle, 1-(3-phénoxy phényl)éthyle ou α-thioamido 3-phénoxy benzyle,

— soit un groupement:

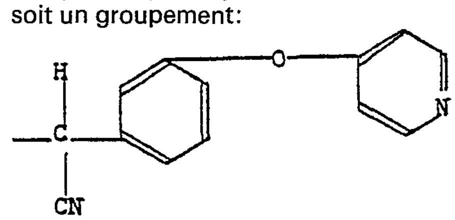

— soit un groupement

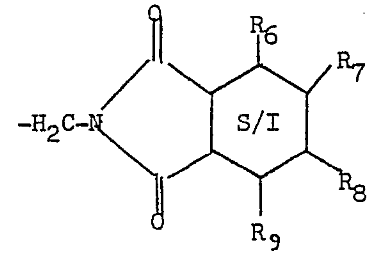

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

– soit un groupement

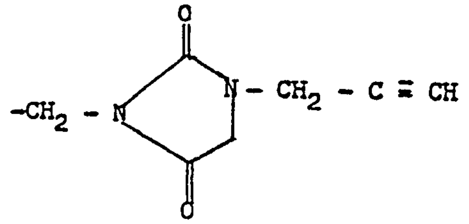

– soit un groupement

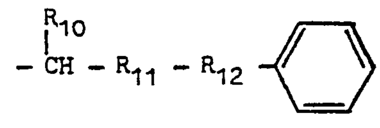

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec $-\underset{R_{10}}{CH}-$ peut se trouver à l'une quelconque des positions disponibles, $R_{12}$ étant lié à $R_{11}$ par l'atome de carbone compris entre l'atome de soufre et un atome d'azote,

– soit un groupement

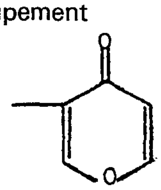

– soit un groupement

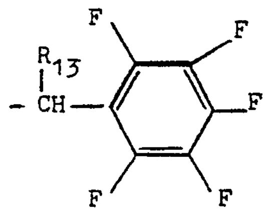

dans lequel $R_{13}$ représente un atome d'hydrogène ou un radical CN,

– soit un groupement

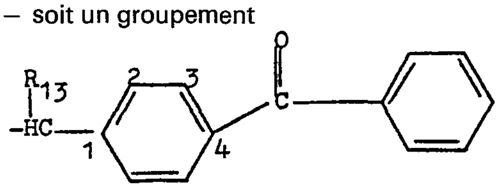

dans lequel $R_{13}$ est défini comme ci-dessus, et le radical benzoyle est en position 4,

– soit un groupement

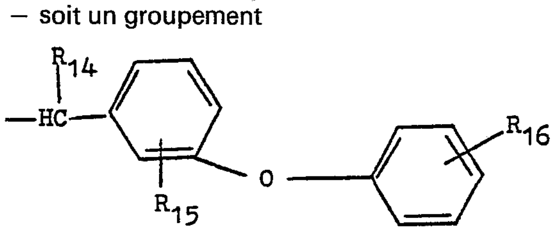

dans lequel $R_{14}$ représente un atome d'hydrogène, un radical méthyle, éthynyle ou cyano, $R_{15}$ représente un atome de fluor de chlore ou de brome et $R_{16}$ représente un atome d'hydrogène, de fluor, de chlore ou de brome,

– soit un groupement

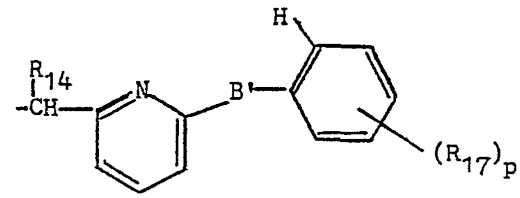

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre et R représente un radical alcoyle renfermant de 1 à 18 atomes de carbone, linéaire, ramifié, saturé ou insaturé, éventuellement substitué par un ou plusieurs groupements fonctionnels identiques ou différents ou bien R représente un radical alcoyle cyclique, saturé ou insaturé, comportant de 3 à 7 atomes de carbone, éventuellement substitué par un ou plusieurs groupements fonctionnels, identiques ou différents, ou bien R représente un groupement aryle, renfermant de 6 à 14 atomes de carbone, éventuellement substitué par un ou plusieurs groupements fonctionnels, identiques ou différents, ou bien R représente un radical hétérocyclique éventuellement substitué par un ou plusieurs groupements fonctionnels identiques ou différents.

Les composés de formule (I) peuvent exister sous de nombreuses formes stéréo-isomères: ils peuvent présenter un ou plusieurs centres d'asymétrie dans la partie A comme dans la partie R.

Lorsque A représente un radical alcoyle, il s'agit de préférence du radical méthyle, éthyle, n-propyle, isopropyle, n-butyle, isobutyle ou terbutyle.

Lorsque A représente un radical benzyle substitué par un ou plusieurs radicaux alcoyle, il s'agit de préférence des radicaux méthyle ou éthyle.

Lorsque A représente un radical benzyle substitué par un ou plusieurs radicaux alcényle, il s'agit de préférence de radicaux vinyle, allyle, 2-méthylallyle ou isobuténylе.

Lorsque A représente un radical benzyle substitué par un ou plusieurs radicaux alcényloxy, il s'agit de préférence de radicaux vinyloxy, allyloxy, 2-méthylallyloxy ou isobuténуloxy.

Lorsque A représente un radical benzyle substitué par un ou plusieurs atomes d'halogène, il s'agit de préférence d'atomes de chlore, de brome ou de fluor.

Le substituant $R_2$ représente notamment un radical phényle.

Le substituant $R_3$ représente de préférence un radical $-CH_2-CH=CH_2$, $-CH_2-CH=CH-CH_3$, $-CH_2CH=CH-CH=CH_2$ ou $-CH_2-CH=CH-CH_2-CH_3$.

Les substituants $R'_1$ et $R'_2$ reeprésentent notamment un atome de fluor, de brome ou de chlore, un radical méthyle, un radical éthyle, un radical hexyle, linéaire ou ramifié, un radical phényle, un groupement méthoxy carbonyle, un groupement éthoxycarbonyle, un groupement pentoxy carbonyle, linéaire ou ramifié.

Le substituant $R_5$ représente notamment un atome de fluor, de chlore ou de brome.

Le substituant $R_{17}$ représente notamment un radical méthyle, éthyle, propyle ou butyle, linéaire ou ramifié, un groupement méthoxy, éthoxy, propoxy ou butoxy, linéaire ou ramifié, un groupement méthylthio, éthylthio, propylthio ou butylthio, linéaire ou ramifié, un groupement méthyl sulfonyle, éthylsulfonyl, propylsulfonyl ou butylsulfonyle, linéaire ou ramifié.

Lorsque R représente un radical alcoyle, linéaire ou ramifié, on entend par alcoyle, par exemple, un radical méthyle, éthyle, propyle, linéaire ou ramifié, butyle, linéaire ou ramifié, pentyle, linéaire ou ramifié, hexyle, linéaire ou ramifié, décyle, linéaire ou ramifié, tétradécyle, linéaire ou ramifié, octadécyle linéaire ou ramifié.

Lorsque R représente un radical alcoyle insaturé, on entend notamment par alcoyle insaturé, un radical éthényle, propényle, butényle, linéaire ou ramifié, penténylе, linéaire ou ramifié, hexényle, linéaire ou ramifié, décényle, linéaire ou ramifié, tétradécényle, linéaire ou ramifié, octadécényle, linéaire ou ramifié, éthynyle, propynyle ou encore des radicaux aliphatiques insaturés comportant deux ou plusieurs doubles liaisons.

Lorsque R représente un radical alcoyle substitué par un plusieurs groupements fonctionnels, on entend de préférence, par alcoyle, un radical renfermant de 1 à 8 atomes de carbone, comme, par exemple, le radical méthyle, éthyle, propyle, linéaire ou ramifié, butyle, linéaire ou ramifié, octyle linéaire ou ramifié.

Lorsque R représente un radical alcoyle substitué par un ou plusieurs groupements fonctionnels, on entend de préférence par groupement fonctionnel, un atome d'hylogène, un groupement OH ou SH, un groupement OR' ou SR' dans lesquels R' représente un radical alcoyle renfermant de 1 à 8 atomes de carbone, un groupement $NO_2$, un groupement:

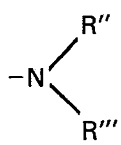

dans lequel R'' et R''', identiques ou différents, représentent un atome d'hydrogène, ou un radical alcoyle renfermant de 1 à 8 atomes de carbone, ou R' représente un groupement: $-C\equiv N$, $SO_3H$ ou $PO_4H_2$ ou un groupement $COalc_1$, $SO_2alc_2$ ou $SO_3alc_3$ dans lesquels $alc_1$, $alc_2$ et $alc_3$ représentent des radicaux alcoyle renfermant de 1 à 18 atomes de carbone. R peut représenter également un radical alcoyle substitué par un radical aryle comme par exemple le radical benzyle ou le radical phénéthyle, lui-même éventuellement substitué par un ou plusieurs groupements OH, Oalc ou alc renfermant de 1 à 8 atomes de carbone, par un ou plusieurs atomes d'halogène, par un ou plusieurs groupements $CF_3$, $OCF_3$, $SCF_3$ ou par un groupement (G):

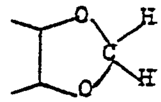

(G)

R peut représenter également un radical alcoyle substitué sur deux carbones adjacents par un groupement ($G_1$)

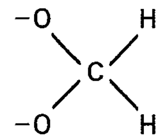

($G_1$)

ou substitué par un groupement

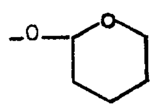

Lorsque R représente un radical alcoyle substitué par un ou plusieurs groupements fonctionnels, on peut citer comme valeurs préférées de R les radicaux:

$-(CH_2)_n-CHal_3$ dans lequel n est un entier de 1 à 8 et Hal un atome d'halogène, par exemple le radical $-CH_2-CCl_3$, $-CH_2-CF_3$, $-CH_2-CH_2-CCl_3$ ou $-CH_2-CH_2-CF_3$,

$-(CH_2)_{n_1}-CH\ Hal_2$ dans lequel Hal est défini comme ci-dessus et $n_1$ est un nombre de 0 à 8, par exemple le radical $-CH_2-CHCl_2$, $-CH_2-CHF_2$ ou $-CHF_2$,

$-(CH_2)_n-CH_2\ Hal$ dans lequel n et Hal sont définis comme ci-dessus, par exemple le radical $-CH_2-CH_2Cl$ ou $-CH_2-CH_2F$,

$-C-(CHal_3)_3$ dans lequel Hal est défini comme ci-dessus, par exemple le radical $-C-(CF_3)_3$ ou

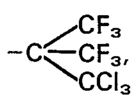, 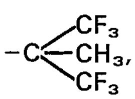, 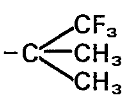 ou

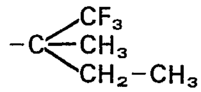 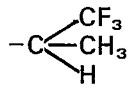 ou 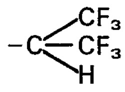

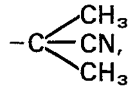, 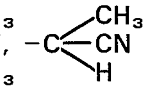 ou $-(CH_2)_n-CN$,

dans lequel n est défini comme précédemment,

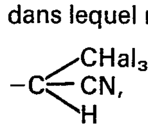 dans lequel Hal est défini comme précédemment,

par exemple le radical 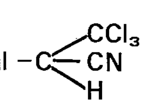

$-(CH_2)_n-OR'$, dans lequel n est défini comme précédemment et R' représente un atome d'hydrogène ou un radical alcoyle linéaire ou ramifié, comportant de 1 à 8 atomes de carbone, par exemple le radical $-CH_2-OCH_3$,
$-CH_2-CH_2-O-CH_3$,
$-CH_2-CH_2-O-CH_2-CH_3$ ou

$-CH_2-CH_2-OH$, 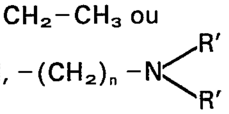

dans lequel n et R' sont définis comme précédemment et les deux radicaux R' peuvent être différents entre eux,

par exemple le radical 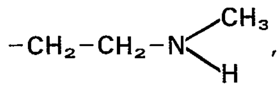 ,

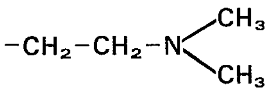 ou

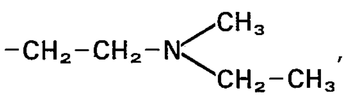 ,

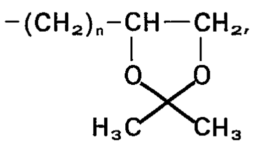

dans lequel n est défini comme précédemment, par exemple le radical

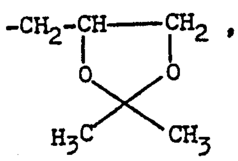 , 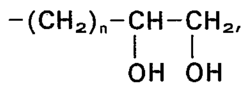

dans lequel n est défini comme précédemment, par exemple le radical 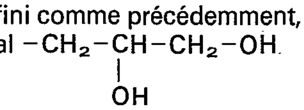

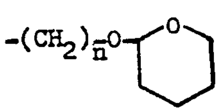

dans lequel n est défini comme précédemment, par exemple le radical

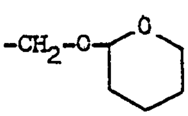 ou 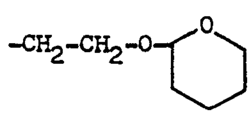

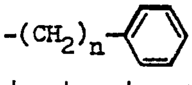

dans lequel n est défini comme précédemment, par exemple le radical benzyle ou phénéthyle,

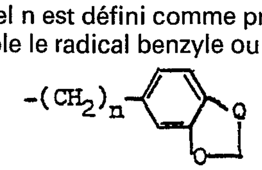

dans lequel n est défini comme précédemment, par exemple le radical

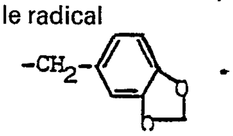 .

Lorsque R représente un radical alcoyle cyclique saturé ou insaturé comportant de 3 à 7 atomes de carbone, il s'agit de préférence d'un cyclopropyle, d'un cyclobutyle, d'un cyclopentyle, d'un cyclohexyle, d'un cycloheptyle ou d'un radical alcoyle substitué par l'un des radicaux cycloalcoyle cidessus ou encore d'un radical 1-méthylcyclobutyle, 1-méthylcyclopentyle, 1-méthylcyclohexyle ou 2,2,3,3-tétraméthylcyclopropyle.

Lorsque R représente un radical alcoyle cyclique comportant de 3 à 7 atomes de carbone, substitué par un ou plusieurs groupements fonctionnels, on entend, de préférence, par groupement fonctionnel, un atome d'halogène, un radical alcoyle comportant de 1 à 6 atomes de carbone, un radical alcoyloxyle comportant de 1 à 6 atomes de carbone, un groupement $NO_2$.

Lorsque R représente un radical aryle éventuellement substitué, il s'agit de préférence du radical phényle ou du radical phényle substitué par un ou plusieurs groupements OH, Oalc ou alc, renfermant de 1 à 8 atomes de carbone par un groupement $CF_3$, $OCF_3$ ou $SCF_3$ ou par un atome de chlore, de brome ou de fluor.

Lorsque R représente un radical hétérocyclique, il s'agit de préférence du radical pyridinyle, furanyle, thiophényle, oxazolyle ou thiazolyle.

L'invention a notamment pour objet les composés de formule (I) tels que définis précédemment, dans laquelle A représente un groupement α-cyano 3-phénoxybenzyle sous forme S, R ou RS, ainsi que ceux pour lesquels A représente un groupement /3-(propyn-2-yl) 2,5-dioxo imidazolidinyl/ méthyle.

L'invention a plus spécialement pour objet les composés de formule (I) tels que définis précédemment, pour lesquels R représente un radical alcoyle, linéaire ou ramifié, renfermant de 1 à 18 atomes de carbone et plus particulièrement ceux pour lesquels R représente un radical méthyle, éthyle, isopropyle ou terbutyle.

L'invention a tout particulièrement pour objet les composés de formule (I) décrits dans les exemples de la partie expérimentale.

L'invention a également pour objet un procédé de préparation des composés de formule (I), caractérisé en ce que l'on fait réagir un acide de formule (II):

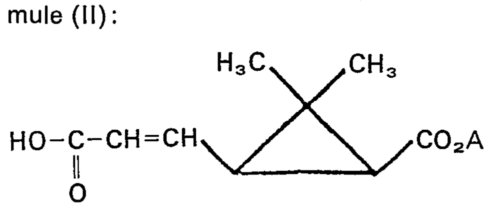 (II)

formule dans laquelle la copule cyclopropanique a la structure 1R cis et la double liaison a la géométrie Z et dans laquelle A conserve les significations précitées, au sein d'un solvant organique, en présence de dicyclohexylcarbodiimide, avec un mercaptan de formule (III):

$$HS-R \qquad (III)$$

formule dans laquelle R conserve les significations précitées.

Le solvant organique au sein duquel on fait réagir le composé de formule (II) avec le mercaptan de formule (III) est choisi notamment dans le

groupe constitué par le chlorure de méthylène, le benzène, le THF.

Il va de soi que si les produits RSH mis en œuvre possèdent des fonctions autres que la fonction SH susceptibles de réagir lors de la réaction avec l'acide (II), il est nécessaire de bloquer les fonctions lors de la condensation. Ces blocages sont effectués par des méthodes connues en elles-mêmes.

La réaction de l'acide (II) avec le mercaptan (III) en présence de dicyclohexyl carbodiimide est effectuée, de préférence, en présence de diméthylamino pyridine.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites des animaux à sang chaud.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et ne sont pas toxiques pour les mammifères.

L'ensemble de ces propriétés fait des produits de formule (I) des produits qui correspondent parfaitement aux exigences de l'industrie agrochimique moderne: ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment comme principe actif au moins un des produits de formule (I) tel que défini précédemment.

L'invention a notamment pour objet les compositions insecticides renfermant comme principe actif au moins l'un des produits de formule (I) tel que défini précédemment.

Parmi les compositions notamment insecticides préférées de l'invention, on peut citer tout spécialement les compositions renfermant un des composés décrits dans les différents exemples de la partie expérimentale.

L'invention a plus spécialement pour objet les compositions insecticides renfermant comme principe actif au moins l'un des produits de formule (I) qui sont décrits dans les exemples de la partie expérimentale.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95% en poids.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide sur végétaux, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrage foliaires contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tels que le 1-(2,5,8-trioxadodécyl) 2-propyl 4,5-méthylène-dioxy benzène (ou butoxyde de pipéronyle) ou la N-(2-éthyl heptyl) bicyclo/2,2-1/5-heptène-2,3-di carboximide, ou le pipéronyl-bis-2-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions destinées à l'alimentation animale renfermant comme principe actif au moins l'un des produits de formule (I) tel que défini précédemment.

Les composés de formule (I) présentent une excellente tolérance générale, et l'invention a donc également pour objet les produits de formule (I), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale. Ils peuvent également être utilisés comme anthelmintiques.

Les médicaments de l'invention peuvent être administrés par voie externe, par vaporisation, par shampooing, par bain ou badigeonnage.

Les médicaments de l'invention à usage vétérinaire peuvent être également administrés par badigeonnage de l'épine dorsale selon la méthode dite méthode «pour-on». Ils peuvent être également administrés par voie digestive ou parentérale.

L'invention a également pour objet les compositions pharmaceutiques renfermant comme principe actif, l'un au moins des médicaments tels que définis précédemment.

On peut indiquer également que les produits de l'invention peuvent être utilisés comme biocides ou comme régulateurs de croissance.

L'invention comprend également les associations douées d'activité insecticide, acaricide ou nématicide, caractérisées en ce qu'elles contiennent comme matière active, d'une part, un au moins des composés de formule générale (I) tels que définis ci-dessus et, d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels «halo» représente un atome de fluor, de chlore ou de brome, étant entendu que les composés (I) peuvent exister sous toutes leurs formes stéréo-isomères possibles, de même que les copules acides et alcools des esters pyréthrinoïdes ci-dessus.

Les associations selon l'invention présentent notamment l'intérêt soit de permettre de combattre, par la polyvalence de leur action, une gamme de parasites plus étendue, soit de manifester, dans certains cas, un effet de synergie.

Les acides (II) utilisés au départ du procédé de préparation des composés de l'invention de formule:

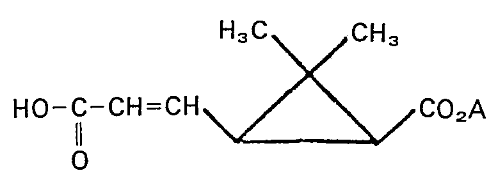

(II)

peuvent être préparés selon les procédés décrits dans les demandes de brevets européens publiées $N^{os}$ 38271, 41021 et 48186.

Les exemples suivants illustrent l'invention sans toutefois la limiter.

*Exemple 1:*

*1R cis 2,2-diméthyl 3/(ΔZ) 3-oxo-3-méthylthio propényl/ cyclopropane carboxylate de S α-cyano-3-phénoxy benzyle et 1R cis 2,2-diméthyl 3/(ΔE) 3-oxo-3-méthylthio propényl/ cyclopropane carboxylate de S α-cyano-3-phénoxy benzyle.*

Dans 20 $cm^3$ de chlorure de méthylène on introduit 4,1 g de 1R cis 2,2-diméthyl 3/(ΔZ) carboxy éthényl/ cyclopropane carboxylate de S(α)-cyano-3-phénoxy benzyle, 50 mg de diméthyl aminopyridine, ajoute à 0° C 2,16 g de dicyclohexyl carbodiimide, agite pendant 5 min, introduit en une seule fois une solution de 545 mg de méthyl mercaptan dans 5 $cm^3$ de benzène, agite pendant 5 min à 0° C, puis pendant 16 heures à 20° C et élimine par filtration l'insoluble formé. On lave le filtrat par une solution aqueuse N d'acide chlorhydrique, à l'eau, concentre le filtrat à sec par distillation sous pression réduite, chromatographie le résidu sur gel de silice en éluant par le mélange cyclohexane-acétate d'éthyle (9/1) et obtient 1,2 g de 1R cis 2,2-diméthyl 3/(ΔZ)3-oxo-3-méthyl thio propényl/ cyclopropane carboxylate de S α-cyano-3-phénoxy benzyle. F=87° C, $[\alpha]_D=+51°$ (c=1% benzène).

*Spectre de RMN (deutérochloroforme)*

– Pics à 1,25-1,27 ppm attribués aux hydrogènes des méthyles géminés.

– Pics à 1,93-2,07 ppm attribués à l'hydrogène en position 1 du cyclopropyle.

– Pic à 2,38 ppm attribué aux hydrogènes du méthyle de $S-CH_3$.

– Pics à 3,2-3,5 ppm attribués à l'hydrogène en position 3 du cyclopropyle.

– Pics à 6,1-6,6 ppm attribués aux hydrogènes éthylémiques.

– Pic à 6,4 ppm attribué à l'hydrogène porté par le carbone en α du groupement CN.

– Pics à 7,0-7,6 ppm attribués aux hydrogènes des noyaux aromatiques.

En continuant la chromatographie, on obtient 1,3 g de 1R cis 2,2-diméthyl 3/(ΔE) 3-oxo-3-méthylthio propényl/cyclopropane carboxylate de S α-cyano 3-phénoxy benzyle, $(\alpha)_D=+67,5°$ (c=0,6% benzène).

*Spectre de RMN (deutérochloroforme)*

– Pics à 1,25-1,28 ppm attribués aux hydrogènes des méthyles géminés.

– Pics à 1,9-2,1 ppm attribués aux hydrogènes en positions 1 et 3 du cyclopropyle.

– Pic à 2,4 ppm attribué aux hydrogènes du méthyle $SCH_3$.

– Pics à 6,2-6,5-7,0-7,2 ppm attribués aux hydrogènes éthyléniques.

– Pic à 6,5 ppm attribué à l'hydrogène porté par le carbone en α du groupement –CN.

– Pics à 7,0 à 7,7 ppm attribués aux hydrogènes des noyaux aromatiques.

Le 1R, cis 2,2-diméthyl 3/(ΔZ) carboxy éthényl/cyclopropane carboxylate de S (α) cyano-3-phénoxy benzyle utilisé au départ de l'exemple 1 peut être préparé comme décrit dans la demande européenne publiée N° 38271.

*Exemple 2:*

*1R cis 2,2-diméthyl 3-/(ΔZ) 3-oxo 3-éthylthio propényl/ cyclopropane carboxylate de S α-cyano 3-phénoxy benzyle.*

Dans 10 $cm^3$ de chlorure de méthylène on introduit 2 g de 1R cis 2,2-diméthyl 3/(ΔZ)carboxy éthényl/ cyclopropane carboxylate de S (α)-cyano-3-phénoxy benzyle, ajoute en une seule fois 1,5 $cm^3$ d'éthane thiol puis à +5° C une solution de 1 g de dicyclohexyl carbodiimide et de 40 mg de diméthylamino pyridine dans 5 $cm^3$ de chlorure de méthylène. On agite pendant 5 min à +5° C et pendant 3 h à température ambiante, élimine par filtration l'insoluble formé, concentre le filtrat à sec par distillation sous pression réduite, chromatographie le résidu sur gel de silice en éluant avec un mélange de n-hexane et d'acétate d'éthyle (8/2) et obtient 1,2 g de 1R cis 2,2-diméthyl 3/(ΔZ) 3-oxo-3-éthylthio propényl/cyclopropane carboxylate de S α-cyano-3-phénoxy benzyle. F=47° C $(\alpha)_D=+59°$ C (c=0,4% chloroforme).

*Spectre de RMN (deutérochloroforme)*

– Pics à 1,25-1,27 ppm attribués aux hydrogènes des méthyles géminés.

– Pics à 1,23-1,31-1,39 ppm, à 2,8-2,9 ppm, à 3,0-3,1 ppm attribués aux hydrogènes du groupement éthyle de $-S-C_2H_5$.

– Pics à 1,95-2,04 ppm attribués à l'hydrogène en position 1 du cyclopropyle.

– Pics à 3,2-3,5 ppm attribués à l'hydrogène en position 3 du cyclopropyle.

– Pics à 6,2-6,5 ppm attribués aux hydrogènes éthyléniques.

– Pics à 7,0-7,6 ppm attribués aux hydrogènes des noyaux aromatiques.

*Exemple 3:*

*1R cis 2,2-diméthyl 3-/(ΔZ) 3-oxo-3-isopropylthio propényl/ cyclopropane carboxylate de (S) α-cyano 3-phénoxy benzyle et 1R cis 2,2-diméthyl 3/(ΔE) 3-oxo-3-isopropylthio propényl/cyclopropane carboxylate de S (α)-cyano-3-phénoxy benzyle.*

Dans une solution de 3,9 g de 1R cis 2,2-diméthyl 3/(ΔZ) carboxy éthényl/ cyclopropane carboxylate de S(α)-cyano-3-phénoxy benzyle dans 25 $cm^3$ de chlorure de méthylène, on ajoute en une fois 3 $cm^3$ de 2-propane thiol, puis à +5° C, 2 g de dicyclohexyl carbodiimide et 70 mg de diméthylamino pyridine en solution dans 10 $cm^3$ de chlorure de méthylène, agite pendant 5 min à +5° C puis pendant 3 h à température ambiante, élimine par filtration l'insoluble formé, concentre le filtrat à sec par distillation sous pression réduite, chromatographie le résidu sur gel de silice en éluant par un mélange de n-hexane et d'acétate d'éthyle (85/15) et obtient 3,1 g de 1R cis 2,2-diméthyl 3/(ΔZ) 3-oxo-3-isopropylthio propényl/ cyclopropane carboxylate de S α-cyano-3-phénoxy benzyle. F=80° C, $(\alpha)_D=+69°$ C (c=0,5% chloroforme).

*Spectre de RMN (deutérochloroforme)*

– Pics à 1,27-1,28 ppm attribués aux hydrogènes des méthyles géminés.

– Pics à 1,3-1,4 ppm attribués aux hydrogènes des méthyles de l'isopropyle.

– Pics à 1,9-2,06 ppm attribués à l'hydrogène en position 1 du cyclopropyle.

– Pics à 3,2-3,5 ppm attribués à l'hydrogène en position 3 du cyclopropyle.

– Pic à 3,7 ppm attribué à l'hydrogène du $-CH-$ de l'isopropyle.

– Pics de 6,1 à 6,6 ppm attribués aux hydrogènes éthyléniques.

– Pic à 6,4 ppm attribué à l'hydrogène porté par le carbone en position α du groupement –CN.

– Pics à 7,0-7,6 ppm attribués aux hydrogènes des noyaux aromatiques.

En continuant la chromatographie on obtient 340 mg de 1R cis 2,2-diméthyl 3/(ΔE) 3-oxo-3-isopropylthio propényl/ cyclopropane carboxylate de S α-cyano 3-phénoxy benzyle. F=87° C $(\alpha)_D = -6{,}5°$ (c=0,5% chloroforme).

*Spectre de RMN (deutérochloroforme)*

– Pics à 1,25-1,29 ppm attribués aux hydrogènes des méthyles géminés.

– Pics à 1,29-1,4 ppm attribués aux hydrogènes des méthyles de l'isopropyle.

– Pics à 1,95-2,16 ppm attribués aux hydrogènes en position 1 et 3 de l'isopropyle.

– Pic à 3,7 ppm attribué à l'hydrogène du $-CH-$ de l'isopropyle.

– Pics à 6,18-6,4 ppm attribués à l'hydrogène éthylénique en α de $-\underset{\underset{O}{\|}}{C}-S-$.

– Pic à 6,5 ppm attribué à l'hydrogène porté par le carbone en α du groupement $-C{\equiv}N$.

– Pics de 7 à 7,7 ppm attribués à l'autre hydrogène éthylénique.

– Pics de 7 à 7,7 ppm attribués aux hydrogènes des noyaux aromatiques.

*Exemple 4:*

*1R cis 2,2-diméthyl 3/(ΔZ) 3-oxo-3-tertbutylthio propényl/ cyclopropane carboxylate de S α-cyano 3-phénoxy benzyle et 1R cis 2,2-diméthyl 3/(ΔE) 3-oxo-3-tertbutylthio propényl/ cyclopropane carboxylate de S α-cyano-3-phénoxy benzyle.*

Dans une solution de 3,46 g de 1R cis 2,2-diméthyl 3/(ΔZ) carboxy éthényl/ cyclopropane carboxylate de S α-cyano 3-phénoxy benzyle dans 20 cm³ de chlorure de méthylène, on introduit à +5° C, 150 mg de diméthyl amino pyridine et 1,9 g de dicyclohexyl carbodiimide, agite à +5° C pendant 5 min, ajoute 5 cm³ de tertbutyl mercaptan, agite pendant 5 min à +5° C, puis pendant 3 h à 20° C, élimine par filtration l'insoluble formé, concentre le filtrat à sec par distillation sous pression réduite, chromatographie le résidu sur gel de silice en éluant par un mélange de cyclohexane et d'acétate d'éthyle (9/1) et obtient 1,4 g de 1R cis 2,2-diméthyl 3/(ΔZ) 3-oxo-3-tertbutylthio propényl/ cyclopropane carboxylate de S α-cyano-3-phénoxy benzyle. F=76° C, $(\alpha)_D = +55°$ C (c=0,5% benzène).

*Spectre de RMN (deutérochloroforme)*

– Pics à 1,25-1,28 ppm attribués aux hydrogènes des méthyles géminés.

– Pic à 1,5 ppm attribué aux hydrogènes du tertbutyle.

– Pics à 1,9-2,1 ppm attribués à l'hydrogène en position 1 du cyclopropyle.

– Pics à 3,2-3,4 ppm attribués à l'hydrogène en position 3 du cyclopropyle.

– Pics à 6,0-6,6 ppm attribués aux hydrogènes éthyléniques.

– Pic à 6,4 ppm attribué à l'hydrogène porté par le carbone en α du groupement –CN.

– Pics de 7,0 à 7,7 ppm attribués aux hydrogènes des noyaux aromatiques.

En continuant la chromatographie on obtient 1,6 g de 1R cis 2,2-diméthyl 3/(ΔE) 3-oxo-3-tertbutylthio propényl/ cyclopropane carboxylate de S α-cyano-3-phénoxy benzyle. F=95° C $(\alpha)_D = +62°$ (c=1% benzène).

*Spectre de RMN (deutérochloroforme)*

– Pics à 1,25-1,30 ppm attribués aux hydrogènes des méthyles géminés.

– Pic à 1,5 ppm attribué aux hydrogènes du tertbutyle.

– Pics à 1,8-2,2 ppm attribués aux hydrogènes en position 1 et 3 du cyclopropyle.

– Pics à 6,2-6,4 ppm attribués à l'hydrogène éthylénique en α de $-\underset{\underset{O}{\|}}{C}-S$ tert Bu.

– Pic à 6,5 ppm attribué à l'hydrogène porté par le carbone en α du groupement –CN.

– Pics de 6,9 à 7,7 ppm attribués à l'autre hydrogène éthylénique et aux hydrogènes des noyaux aromatiques.

*Exemple 5:*

*1R cis 2,2-diméthyl 3/(ΔZ) 3-oxo-3-thio méthyl propényl/ cyclopropane carboxylate de 3(propyn-2-yl) 2,5-dioxo imidazolidinyl-méthyle.*

Dans 5 cm³ de chlorure de méthylène, on introduit 2,3 g de 1R cis 2,2-diméthyl 3/(ΔZ) carboxy éthényl/ cyclopropane carboxylate de 3(propyn-2-yl) 2,5-dioxo imidazolidinyl méthyle, 2,2 g de méthyl mercaptan en solution dans 5 cm³ de chlorure de méthylène, ajoute à +5° C une solution de 1,45 g de dicyclohexylcarbodiimide et de 40 mg de diméthyl amino pyridine dans 5 cm³ de chlorure de méthylène, agite pendant 5 min à +5° C puis pendant 3 h à température ambiante. On élimine par filtration l'insoluble formé, concentre le filtrat à sec par distillation sous pression réduite, chromatographie le résidu sur gel de silice en éluant par un mélange de n-hexane et d'acétate d'éthyle (65/35) et obtient 2,3 g de 1R cis 2,2-diméthyl 3/(ΔZ) 3-oxo-3-thio méthyl propényl/ cyclopropane carboxylate de 3-(propyn-2-yl) 2,5-dioxo imidazolidinyl méthyle. $(\alpha)_D = +17°$ (c=0,5% chloroforme).

*Spectre de RMN (deutérochloroforme)*

– Pics à 1,26-1,30 ppm attribués aux hydrogènes des méthyles géminés.

– Pics à 1,8-2,0 ppm attribués à l'hydrogène en position 1 du cyclopropyle.

– Pics à 2,33-2,37-2,42 ppm attribués à l'hydrogène de l'éthynyle.

– Pic à 2,35 ppm attribué aux hydrogènes du méthyle du $-SCH_3$.

– Pics à 3,1-3,4 ppm attribués à l'hydrogène en position 3 du cyclopropyle.

– Pic à 4,0 ppm attribué à l'hydrogène en position 4 du noyau imidazolidinyle.

– Pics à 4,25-4,29 ppm attribués aux hydrogènes du méthylène du propynyle situé en α du noyau imidazolidinyle.

– Pics à 5,3-5,5 et 5,5-5,7 ppm attribués aux hydrogènes du méthylène en α de $-\underset{\underset{O}{\|}}{C}-O-$.

– Pics à 6,1-6,3 ppm attribués à l'hydrogène éthylénique en α de $-\underset{\underset{O}{\|}}{C}-S-CH_3$.

– Pics à 6,2-6,6 ppm attribués à l'autre hydrogène éthylénique.

Le 1 R cis 2,2-diméthyl 3/(ΔZ) carboxy éthényl/ cyclopropane carboxylate de 3(propyn-2-yl) 2,5 dioxo imidazolidinyl méthyle utilisé au départ de l'exemple 5 peut être préparé comme suit:

Dans 35 $cm^3$ de toluène, on introduit 3,7 g de 1R, cis 2,2-diméthyl 3/(ΔZ) 3-oxo-3-terbutoxy propényl/cyclopropane carboxylate de 1-/3-(propyn 2-yl)2,5-dioxo imidazolidinyl méthyle/ (décrit dans la demande européenne publiée N° 41021), ajoute 300 mg d'acide paratoluène sulfonique monohydraté, porte le mélange réactionnel au reflux et l'y maintient jusqu'à cessation de dégagement gazeux, refroidit à 0° C, élimine par filtration l'insoluble résiduel, concentre à sec le filtrat par distillation sous pression réduite, chromatographie le résidu sur silice en éluant avec un mélange d'hexane, d'acétate d'éthyle et d'acide acétique (60/40/1) et obtient 3,1 g de 1R, cis 2,2-diméthyl 3/(ΔZ) carboxy éthényl cyclopropane carboxylate de 3-(propyn-2-yl)2,5 imidazolidinyl méthyle. $(\alpha)_D=+16°$ (c=0,25% chloroforme).

*Exemple 6:*

*1R, cis 2,2-diméthyl 3/(ΔZ) 3-oxo-3-éthylthio propényl/cyclopropane carboxylate de /3(propyn-2-yl)2,5-dioxo imidazolidinyl/-méthyle.*

En opérant de manière analogue à celle de l'exemple 5 au départ de 2,1 g de 1R, cis 2,2-diméthyl 3/(ΔZ) carboxy éthényl/cyclopropane carboxylate de 3(propyn-2-yl)2,5-dioxo imidazolidinyl méthyle et d'éthyl mercaptan, après chromatographie sur silice en éluant avec un mélange de n-hexane et d'acétate d'éthyle (6/4), on obtient 1,57 g de 1R, cis 2,2-diméthyl 3/(ΔZ) 3-oxo 3-éthylthio propényl/cyclopropane carboxylate de /3(propyn-2-yl)2,5-dioxo imidazolidinyl/méthyle. $(\alpha)_D=+19,5°$ C (c=0,5% chloroforme).

*Spectre de RMN (deutérochloroforme)*

– Pics à 1,26-1,30 ppm attribués aux hydrogènes des méthyles géminés.

– Pics à 1,18-1,30-1,42 ppm attribués aux hydrogènes de l'éthyle de $-SC_2H_5$.

– Pics à 1,84-1,98 ppm attribués à l'hydrogène en position 1 du cyclopropyle.

– Pics à 2,34-2,38-2,42 ppm attribués à l'hydrogène du ≡CH de l'éthynyle.

– Pics à 3,13-3,4 ppm attribués à l'hydrogène en position 3 du cyclopropyle.

– Pic à 4,0 ppm attribué aux hydrogènes en position 4 du noyau pyrimidinyle.

– Pics à 4,30-4,32 ppm attribués aux hydrogènes du méthylène en α du groupement éthényl.

– Pic à 5,6 ppm attribué aux hydrogènes en α du $-\underset{\underset{O}{\|}}{C}-O-$.

– Pics à 6,1-6,6 ppm attribués aux hydrogènes éthyléniques.

*Exemple 7:*

*1R cis 2,2-diméthyl 3/(ΔZ)3-oxo-3-isopropylthio propényl/ cyclopropane carboxylate de 3(propyn-2-yl) 2,5-dioxo imidazolidinyl méthyle.*

En opérant de façon analogue à celle de l'exemple 5, au départ de 1 g de 1R, cis 2,2-diméthyl 3/ (ΔZ) carboxy éthényl/cyclopropane carboxylate de 3(propyn-2-yl)2,5-dioxo imidazolidinyl méthyle et de 2-propane thiol, après chromatoraphie sur silice en éluant avec un mélange de n-hexane et d'acétate d'éthyle (7/3), on obtient 750 mg de 1R, cis 2,2-diméthyl 3/(ΔZ) 3-oxo 3-isopropylthio propényl/cyclopropane carboxylate de 3 (propyn-2-yl)2,5-dioxo imidazolidinyl méthyle. $(\alpha)_D=+28°$ C (c=0,4% chloroforme).

*Spectre de RMN (deutérochloroforme)*

– Pics à 1,26-1,28 ppm attribués aux hydrogènes des méthyles géminés.

– Pics à 1,3-1,4 ppm attribués aux hydrogènes des méthyles de l'isopropyle.

– Pics à 1,8-2,0 ppm attribués à l'hydrogène en position 1 du cyclopropyle.

– Pics à 2,31-2,36-2,40 ppm attribués à l'hydrogène du ≡CH de l'éthynyle.

– Pics à 3,1-3,4 ppm attribués à l'hydrogène en position 3 du cyclopropyle.

– Pic à 3,7 ppm attribué à l'hydrogène de $-CH<$ de l'isopropyle.

– Pic à 4,0 ppm attribué aux hydrogènes du méthylène en position 4 du cycle imidazolidinyle.

– Pics à 4,25-4,29 ppm attribués à l'hydrogène du méthylène en position α de l'éthynyle.

– Pic à 5,5 ppm attribué aux hydrogènes du méthylène en α du $-\underset{\underset{O}{\|}}{C}-O-$.

– Pics à 6-6,2 ppm attribués à l'hydrogène éthylénique en α de $-\underset{\underset{O}{\|}}{C}-S-\!\!<$

– Pics à 6,2-6,6 ppm attribués à l'autre hydrogène éthylénique.

*Exemple 8:*

*1R, cis 2,2-diméthyl 3/(ΔZ) 3-oxo-3-tert butylthio propényl/cyclopropane carboxylate de 3-(propyn-2-yl)2,5-dioxo imidazolidinyl méthyle et 1R, cis 2,2-diméthyl 3/(ΔE) 3-oxo 3-tert butylthio propényl/cyclopropane carboxylate de 3(propyn-2-yl) 2,5-dioxo imidazolidinyl méthyle.*

En opérant de façon analogue à celle de l'exemple 5 au départ de 1,65 g de 1R, cis 2,2-diméthyl 3/(ΔZ) carboxy éthényl/cyclopropane carboxylate de 3(propyn-2-yl) 2,5-dioxo imidazolidinyl méthyle et de tert butyl mercaptan, après chromatographie sur silice en éluant avec un mélange de n-hexane et d'acétate d'éthyle (6/4) puis avec un mélange de n-hexane et d'acétate d'éthyle (7/3), on obtient 900 mg de 1R, cis 2,2-diméthyl 3/(ΔZ)3-oxo-3-tertbutylthio propényl/cyclopropane carboxylate de 3(propyn-2-yl)2,5-dioxo imidazolidinyl méthyle. $(\alpha)_D=+46{,}5°$ (c=0,5% chloroforme).

*Spectre de RMN (deutérochloroforme)*

– Pics à 1,27-1,32 ppm attribués aux hydrogènes des méthyles géminés.

– Pic à 1,5 ppm attribué aux hydrogènes du tertbutyle.

– Pics à 1,83-1,97 ppm attribués à l'hydrogène en position 1 du cyclopropyle.

– Pics à 2,32-2,35-2,40 ppm attribués à l'hydrogène de ≡CH de l'éthynyle.

– Pics à 3,12-3,4 ppm attribués à l'hydrogène en position 3 du cyclopropyle.

– Pic à 4,05 ppm attribué aux hydrogènes du méthylène en position 4 du noyau imidazolidinyl.

– Pics à 4,25-4,29 ppm attribués aux hydrogènes du méthylène en α de l'éthynyle.

– Pic à 5,5 ppm attribué aux hydrogènes du méthylène en α de $-\underset{\underset{O}{\|}}{C}-O-$.

– Pics à 5,9-6,1 ppm attribués à l'hydrogène éthylénique en α de $-\underset{\underset{O}{\|}}{C}-S$ .

– Pics à 6,1-6,5 ppm attribués à l'autre hydrogène éthylénique.

En poursuivant la chromatographie on obtient 900 mg de 1R cis 2,2-diméthyl 3/(ΔE) 3-oxo-3-tertbutylthio propényl/cyclopropane carboxylate de 3(propyn-2-yl) 2,5-dioxo imidazolidinyl méthyle. $(\alpha)_D=-74{,}5°$ C (c=0,5% chloroforme).

*Spectre de RMN (deutérochloroforme)*

– Pics à 1,22-1,34 ppm attribués aux hydrogènes des méthyles géminés.

– Pic à 1,5 ppm attribué aux hydrogènes du tertbutyle.

– Pics de 1,67 à 2,0 ppm attribués aux hydrogènes en position 1 et 3 du cyclopropyle.

– Pics à 2,32-2,37-2,41 ppm attribués à l'hydrogène du ≡CH de l'éthynyle.

– Pic à 4,05 ppm attribué aux hydrogènes du méthylène en position 4 du noyau imidazolidinyle.

– Pics à 4,25-4,29 ppm attribués aux hydrogènes du méthylène en position α de l'éthynyle.

– Pics à 5,36-5,52 ppm et 5,53-5,68 ppm attribués aux hydrogènes du méthylène en α du $-\underset{\underset{O}{\|}}{C}-O-$.

– Pics à 6,0-6,3 ppm attribués à l'hydrogène en α de $-\underset{\underset{O}{\|}}{C}-S-$.

– Pics à 6,8-7,3 ppm attribués à l'autre hydrogène éthylénique.

*Exemple 9:*

*1R, cis 2,2-diméthyl 3-/(ΔZ) 3-oxo 3-isopropylthiopropényl/cyclopropane carboxylate de (RS) cyano 2-(6-phénoxy pyridyl) méthyle et 1R, cis 2,2-diméthyl 3-/(ΔE) 3-oxo 3-isopropyl thio propényl/cyclopropane carboxylate de (RS) cyano 2-(6-phénoxy pyridyl)méthyle.*

On opère de manière analogue à celle décrite à l'exemple 5, en partant de 4,1 g de 1R, cis 2,2-diméthyl 3/(ΔZ) carboxy éthényl/cyclopropane carboxylate de (RS) cyano 2-(6-phénoxy pyridyl)méthyle et d'isopropylmercaptan et obtient après chromatographie sur silice en éluant au chlorure de méthylène

– 1,9 g d'isomère Z attendu, $(\alpha)_D^{20}=+61{,}5°\pm2{,}5°$ C (c=0,5% benzène).

*Analyse* pour $C_{25}H_{26}N_2O_4S$ (450,561)

Calculé: C 66,65 H 5,82 N 6,22 S 7,12%

Trouvé: C 66,7 H 5,9 N 6,2 S 7,0 %

– 1,2 g d'isomère E attendu, $(\alpha)_D^{20}=-45°$ (c=0,25% benzène)

*Analyse* pour $C_{25}H_{26}N_2O_4S$ (450,561)

Trouvé: C 66,3 H 5,8 N 6,1 S 7,2%

L'acide de départ est décrit dans la demande de brevet européenne publiée N° 0041021.

*Exemple 10:*

*1R, cis 2,2-diméthyl 3/(ΔZ) 3-oxo 3-tertbutylthio propényl/cyclopropane carboxylate de (RS) cyano 2-(6-phénoxy pyridyl)méthyle et 1R, cis 2,2-diméthyl 3/(ΔE) 3-oxo 3-tert-butyl thio propényl/cyclopropane carboxylate de (RS) cyano 2-(6-phénoxy pyridyl)méthyle.*

On opère de manière analogue à celle décrite à l'exemple 9 en utilisant le tertbutyl mercaptan et obtient au départ de 4 g d'acide

– 1,5 g d'isomère Z attendu, $(\alpha)_D^{20}=+71{,}5°\pm3°$ (c=0,5% benzène).

*Analyse* pour $C_{26}H_{28}N_2O_4S$ (464,588)

Calculé: C 67,22 H 6,08 N 6,03 S 6,90%

Trouvé: C 66,9 H 6,0 N 5,9 S 6,9 %

– 1,8 g d'isomère E attendu $(\alpha)_D^{20}=-41°\pm2°$ (c=0,9% benzène).

*Analyse* pour $C_{26}H_{28}N_2O_4S$ (464,588)
Trouvé: C 66,2 H 6,1 N 5,8 S 6,5%

*Exemple 11:*

*1R, cis 2,2-diméthyl 3/(ΔZ) 3-oxo 3-éthylthio propényl/cyclopropane carboxylate de (RS) cyano 2-(6-phénoxy pyridyl)méthyle et 1R, cis 2,2-diméthyl 3/(ΔE) 3-oxo 3-éthylthio propényl/cyclopropane carboxylate de (RS) cyano 2-(6-phénoxy pyridyl)méthyle.*

On opère de manière analogue à celle décrite à l'exemple 9, en utilisant l'éthyl mercaptan et obtient au départ de 4 g d'acide,

– 2,62 g d'isomère Z attendu, $(\alpha)_D^{20}$= +94,5°±2,5° (c=1% dans le chloroforme).

*Analyse* pour $C_{26}H_{24}N_2O_4S$ (436,534)
Calculé: C 66,04 H 5,54 N 6,42 S 7,34%
Trouvé: C 66,3 H 5,6 N 6,1 S 7,6 %

– 0,64 g d'isomère E attendu, $(\alpha)_D^{20}$= −58° ±2,5° (c=0,5% dans le chloroforme).

*Analyse:*
Trouvé: C 66,0 H 5,8 N 6,1 S 7,1%

*Exemple 12:*

*1R, cis 2,2-diméthyl 3/(ΔZ) 3-oxo 3-méthylthio propényl/cyclopropane carboxylate de (RS) cyano 2-(6-phénoxy pyridyl)méthyle et 1R, cis 2,2-diméthyl 3/(ΔE) 3-oxo 3-méthylthio propényl/cyclopropane carboxylate de (RS) cyano 2-(6-phénoxy pyridyl)méthyle.*

On opère de manière analogue à celle décrite à l'exemple 9, en utilisant le méthylmercaptan et en purifiant les produits par chromatographie sur silice en éluant au mélange hexane acétate d'éthyle (8-2). On obtient, au départ de 3,4 g d'acide,

– 1,42 g d'isomère Z attendu $(\alpha)_D^{20}$=+71,5°±3° (c=0,5% $CHCl_3$).

*Analyse* pour $C_{23}H_{22}N_2O_4S$ (422,506)
Calculé: C 65,38 H 5,25 N 6,63 S 7,59%
Trouvé: C 65,3 H 5,4 N 6,4 S 7,6 %

– 0,76 g d'isomère E attendu $(\alpha)_D^{20}$= −50,5°±2° (c=0,5% $CHCl_3$).

*Analyse:*
Trouvé: C 65,3 H 5,5 N 6,4 S 7,6%

*Exemple 13:*

*1R, cis 2,2-diméthyl 3/(ΔZ) 3-oxo 3-méthylthio propényl/cyclopropane carboxylate de (S) cyano 3-phénoxy 4-fluorophényl méthyle.*

On mélange 2,7 g de chlorure de 1R, cis 2,2-diméthyl 3-/(ΔZ) carboxyéthényl/cyclopropane carboxylate de (S) cyano 3-phénoxy 4-fluorophénylméthyle, 15 cm³ de chlorure de méthylène et 4 g de carbonate de calcium et introduit une solution de 1,73 g de méthylmercaptan dans 4 cm³ de benzène. On agite 60 h à 20°C, filtre, lave le filtrat avec une solution aqueuse de phosphate monosodique, puis à l'eau, sèche et concentre à sec. On chromatographie sur silice en éluant au chlorure de méthylène, on obtient 1,3 g de produit attendu. F=103°C $(\alpha)_D^{20}$=+55°±2° (c=0,8% benzène).

*Analyse* pour $C_{24}H_{22}FNO_4S$ (439,510)
Calculé: C 65,59 H 5,05 F 4,32 N 3,19 S 7,30%
Trouvé: C 65,7 H 5,0 F 4,4 N 3,2 S 7,1 %

Le chlorure d'acide de départ peut être obtenu selon des méthodes connues, à partir de l'acide correspondant, lui-même pouvant être obtenu selon le procédé décrit dans la demande européenne publiée N° 0041021.

*Exemple 14:*

*1R, cis 2,2-diméthyl 3-(ΔZ) 3-oxo 3-isopropylthio propényl/cyclopropane carboxylate de (S) cyano 3-phénoxy 4-fluoro phényl méthyle.*

On opère de manière analogue à celle décrite à l'exemple 5, en utilisant au départ 2,9 g de 1R, cis 2,2-diméthyl 3-/(ΔZ) carboxyéthényl/cyclopropane carboxylate de (S)cyano 3-phénoxy 4-fluoro phényl méthyle et l'isopropylmercaptan. On obtient après chromatographie sur silice en éluant au mélange hexane-acétate d'éthyle (8/2), 1,2 g de produit attendu. $(\alpha)_D^{20}$=+55°±2,5° (c=0,6% benzène). F=64°C.

*Analyse* pour $C_{26}H_{26}FNO_4S$ (467,564)
Calculé: C 66,79 H 5,60 F 4,06 N 3,00 S 6,86%
Trouvé: C 67,1 H 5,6 F 4,3 N 3,0 S 6,9 %

L'acide de départ peut être obtenu selon le procédé décrit dans la demande européenne publiée N° 0041021.

*Exemple 15:*

*1R, cis 2,2-diméthyl 3-/(ΔZ) 3-oxo 3-éthylthio propényl/cyclopropane carboxylate de (S) cyano 3-phénoxy 4-fluoro phényl méthyle.*

On opère de manière analogue à celle décrite à l'exemple 14, en utilisant l'éthylmercaptan. On obtient le produit attendu. $(\alpha)_D^{20}$=+58°±2,5° (c=0,6% benzène).

*Analyse* pour $C_{25}H_{24}FNO_4S$ (453,537)
Calculé: C 66,21 H 5,33 F 4,19 N 3,09 S 7,07%
Trouvé: C 66,2 H 5,3 F 4,5 N 3,0 S 7,0 %

*Exemple 16:*

*1R, cis 2,2-diméthyl 3-/(ΔZ) 3-oxo 3-tertbutylthio propényl/cyclopropane carboxylate de (S) cyano 3-phénoxy 4-fluorophénylméthyle et 1R, cis 2,2-diméthyl 3-/(ΔE) 3-oxo 3-tertbutylthio propényl/cyclopropane carboxylate de (S) cyano 3-phénoxy 4-fluorophénylméthyle.*

On opère de manière analogue à celle décrite à l'exemple 14, en utilisant le tertbutylmercaptan. On obtient au départ de 2,7 g d'acide:

– 0,61 g d'isomère Z attendu. F=96°C $(\alpha)_D^{20}$= +63,5°±2,5° (c=0,75% benzène).

*Analyse* pour $C_{27}H_{28}FNO_4S$ (481,591)

Calculé: C 67,34 H 5,86 F 3,94 N 2,91 S 6,66%

Trouvé: C 67,5 H 5,9 F 3,9 N 2,8 S 6,6 %

– 3,4 g d'isomère E attendu. F=122° C $(\alpha)_D^{20}=+61,5°\pm2,5°$ (c=0,5% benzène).

*Analyse:*

Trouvé: C 67,4 H 5,8 F 4,1 N 2,8 S 6,4%

*Exemple 17:*

*1R, cis 2,2-diméthyl 3-/(ΔZ) 3-oxo 3-méthylthio propényl/cyclopropane carboxylate de (1S) 2-méthyl 4-oxo 3-(2-propényl)-2-cyclopentén-1-yle.*

On mélange 2,6 g de 1R, cis 2,2-diméthyl 3-/(ΔZ) carboxy éthényl/cyclopropane carboxylate de (1S) 2-méthyl 4-oxo 3-(2-propényl)-2-cyclopentén-1-yle, 26 cm³ de chlorure de méthylène et 473 mg de méthylmercaptan. On refroidit à 0 à 5° C et ajoute 260 mg de 4-diméthylaminopyridine et 1,7 g de dicyclohexylcarbodiimide en solution dans 10 cm³ de chlorure de méthylène. On maintient 10 min à 0 à +5° C puis 1 h à 20° C. On filtre, lave le filtrat à l'acide chlorhydrique 1N puis à l'eau, sèche et évapore le solvant. On obtient 2,6 g de produit brut que l'on chromatographie sur silice en éluant au mélange hexane-acétate d'éthyle (8-2). On obtient 1,5 g de produit attendu $(\alpha)_D^{20}=+97°\pm2,5°$ (c=0,5% $CHCl_3$).

*Analyse* pour $C_{19}H_{24}O_4S$ (348,465)

| | | | |
|---|---|---|---|
| Calculé: | C 65,49 | H 6,94 | S 9,20% |
| Trouvé: | C 65,8 | H 7,0 | S 9,2 % |

L'acide de départ peut être obtenu selon le procédé décrit dans la demande européenne publiée N° 0041021.

*Exemple 18:*

*1R, cis 2,2-diméthyl 3-/(ΔZ) 3-oxo 3-éthylthio propényl/cyclopropane carboxylate de (1S) 2-méthyl 4-oxo 3-(2-propényl)-2-cyclopentén-1-yle.*

On opère de manière analogue à celle décrite à l'exemple 17, en utilisant l'éthylmercaptan. On obtient, au départ de 2,5 g d'acide, 1,055 g de produit attendu. $(\alpha)_D^{20}=+91°\pm1,5°$ (c=1,2% $CHCl_3$)

*Analyse* pour $C_{20}H_{20}O_4S$ (362,492)

| | | | |
|---|---|---|---|
| Calculé: | C 66,37 | H 7,23 | S 8,85% |
| Trouvé: | C 66,4 | H 7,4 | S 8,6 % |

*Exemple 19:*

*1R, cis 2,2-diméthyl 3-/(ΔZ) 3-oxo 3-isopropylthio propényl/cyclopropane carboxylate de 3-phénoxyphényl méthyle et 1R, cis 2,2-diméthyl 3-/(ΔE) 3-oxo 3-isopropylthiopropényl/cyclopropane carboxylate de 3-phénoxy phényl méthyle.*

On mélange 2,5 g de 1R, cis 2,2-diméthyl 3-/(ΔZ) carboxyéthényl/cyclopropane carboxylate de 3-phénoxyphényl méthyle et 10 cm³ de chlorure de méthylène, refroidit à +5° C, ajoute 1 cm³ d'isopropyl mercaptan, 25 mg de 4-diméthylamino pyridine et 1,41 g de dicyclohexylcarbodiimide dans 10 cm³ de chlorure de méthylène. On agite 10 min à 10° C et 4 h à 20° C, filtre, lave le filtrat à l'eau, sèche, évapore à sec et chromatographie le résidu sur silice en éluant au mélange hexane-acétate d'éthyle (85-15). On obtient:

– 1,413 g d'isomère Z attendu. $(\alpha)_D^{20}=+65°\pm1,5°$ (c=0,7% $CHCl_3$).

*Analyse* pour $C_{25}H_{28}O_4S$ (424,564)

| | | | |
|---|---|---|---|
| Calculé: | C 70,73 | H 6,65 | S 7,55% |
| Trouvé: | C 71,0 | H 6,8 | S 7,6 % |

– 1,003 g d'isomère E attendu. $(\alpha)_D^{20}=-62°\pm2,5°$ (c=0,5% $CHCl_3$).

*Analyse:*

Trouvé: C 71,0 H 6,8 S 7,4%

L'acide de départ peut être préparé selon le procédé décrit dans la demande européenne publiée N° 0041021.

*Exemple 20:*

*1R, cis 2,2-diméthyl 3-/(ΔZ) 3-oxo 3-éthylthio propényl/cyclopropane carboxylate de 3-phénoxyphényl méthyle.*

On opère de manière analogue à celle décrite à l'exemple 19, en utilisant l'éthylmercaptan et obtient, au départ de 2,5 g d'acide, 2,14 g de produit attendu. $(\alpha)_D^{20}=+64,5°\pm2,5°$ (c=0,5% $CHCl_3$).

*Analyse* pour $C_{24}H_{26}O_4S$ (410,536)

| | | | |
|---|---|---|---|
| Calculé: | C 70,22 | H 6,38 | S 7,81% |
| Trouvé: | C 70,5 | H 6,4 | S 7,7 % |

*Exemple 21:*

*1R, cis 2,2-diméthyl 3-/(ΔZ) 3-oxo 3-méthylthio propényl/cyclopropane carboxylate de 3-phénoxy phénylméthyle.*

On opère de manière analogue à celle décrite à l'exemple 19, en utilisant le méthylmercaptan et obtient, au départ de 2,1 g d'acide, 1,82 g de produit attendu. $(\alpha)_D^{20}=+60,5°\pm3°$ (c=0,7% $CHCl_3$).

*Analyse* pour $C_{23}H_{24}O_4S$ (396,509)

| | | | |
|---|---|---|---|
| Calculé: | C 69,67 | H 6,10 | S 8,09% |
| Trouvé: | C 69,6 | H 6,1 | S 7,8 % |

*Exemple 22:*

*1R, cis 2,2-diméthyl 3-/(ΔZ) 3-oxo 3-tertbutylthio propényl/cyclopropane carboxylate de 3-phénoxyphénylméthyle et 1R, cis 2,2-diméthyl 3-/(ΔE) 3-oxo 3-tertbutylthio propényl/cyclopropane carboxylate de 3-phénoxyphényl méthyle.*

On opère de manière analogue à celle décrite à l'exemple 19, en utilisant le tertbutylmercaptan et obtient, au départ de 2,5 g d'acide:

– 0,2 g d'isomère Z attendu. $(\alpha)_D^{20}=+73°\pm3°$ (c=0,5% $CHCl_3$).

*Analyse* pour $C_{26}H_{30}SO_4$ (438,591)

| | | | |
|---|---|---|---|
| Calculé: | C 71,20 | H 6,89 | S 7,31% |
| Trouvé: | C 71,3 | H 7,1 | S 7,5 % |

– 0,7 g d'isomère E attendu $(\alpha)_D^{20}=-65°\pm2,5°$ (c=0,5% $CHCl_3$)

*Analyse:*

Trouvé: C 70,7 H 7,0 S 6,9%

*Exemple 23:*

*1R, cis 2,2-diméthyl 3-/(ΔZ) 3-oxo 3-tertbutylthio propényl/cyclopropane carboxylate de (R) α-(3-phénoxyphényl) éthyle et 1R, cis 2,2-diméthyl 3-/(ΔE) 3-oxo 3-tertbutylthio propényl/cyclopropane carboxylate de (R) α-(3-phénoxyphényl)éthyle*

On opère de manière analogue à celle décrite à l'exemple 19, en utilisant le tertbutylmercaptan et 3 g de 1R, cis 2,2-diméthyl 3-(ΔZ) carboxyéthényl/cyclopropane carboxylate de (R) α-(3-phénoxyphényl)éthyle et obtient, après chromatographie sur silice en éluant au mélange hexane-acétate d'éthyle (95-5),

– 0,97 g d'isomère Z attendu $(\alpha)_D^{20}=+134°\pm3,5°$ (c=0,65% $CHCl_3$)

*Analyse* pour $C_{27}H_{32}O_4S$ (452,618)

| | | | |
|---|---|---|---|
| Calculé: | C 71,65 | H 7,13 | S 7,08% |
| Trouvé: | C 71,5 | H 7,2 | S 7,1 % |

– 1,10 g d'isomère E attendu $(\alpha)_D^{20}=+44,5°\pm1,5°$ (c=0,85% $CHCl_3$)

*Analyse:*

Trouvé: C 71,3 H 7,2 S 7,0%

L'acide de départ est décrit dans la demande de brevet européen publiée N° 0041021.

*Exemple 24:*

*1R, cis 2,2-diméthyl 3-/(ΔZ) 3-oxo 3-tertbutylthio propényl/cyclopropane carboxylate de (3-phénoxy 4-fluoro)phényl méthyle et 1R, cis 2,2-diméthyl 3-/(ΔE) 3-oxo 3-tertbutylthio propényl/cyclopropane carboxylate de (3-phénoxy 4-fluoro) phénylméthyle*

On opère de manière analogue à celle décrite à l'exemple 19, en utilisant le tertbutylmercaptan et 9,7 g de 1R, cis 2,2-diméthyl 3-/(ΔZ)carboxy-éthényl/cyclopropane carboxylate de (3-phénoxy 4-fluoro)phényl méthyle et obtient après chromatographie sur silice en éluant au mélange hexane-acétate d'éthyle (95-5):

– 1,96 g d'isomère Z attendu. $(\alpha)_D^{20}=+80,5°\pm3°$ (c=0,5% $CHCl_3$).

*Analyses* pour $C_{26}H_{29}FO_4S$ (456,581)

| | | | | |
|---|---|---|---|---|
| Calculé: | C 68,40 | H 6,40 | F 4,16 | S 7,02% |
| Trouvé: | C 68,5 | H 6,6 | F 4,1 | S 7,1 % |

– 8,5 g d'isomère E attendu. $(\alpha)_D^{20}=-62°\pm2,5°$ (c=0,6% $CHCl_3$). F=82° C.

*Analyse:*

Trouvé: C 68,4 H 6,6 F 4,1 S 7,1%

L'acide de départ peut être préparé selon le procédé décrit dans la demande de brevet européen publiée N° 0041021.

*Exemple 25:*

*1R, cis 2,2-diméthyl 3-/(ΔZ) 3-oxo 3-méthyl-thio propényl/cyclopropane carboxylate de pentafluorophénylméthyle.*

On opère de manière analogue à celle décrite à l'exemple 19, en utilisant le méthylmercaptan et 2,6 g de 1R, cis 2,2-diméthyl 3-/(ΔZ)carboxy-éthényl/cyclopropane carboxylate de pentafluorophénylméthyle et obtient, après chromatographie sur silice en éluant au mélange hexane-acétate d'éthyle (95-5), 1,9 g de produit attendu. F=45° C. $(\alpha)_D^{20}=+36°\pm1,5°$ (c=1% $CHCl_3$).

*Analyse* pour $C_{17}H_{15}F_5O_3S$ (394,364)

| | | | | |
|---|---|---|---|---|
| Calculé: | C 51,78 | H 3,83 | F 24,09 | S 8,13% |
| Trouvé: | C 51,9 | H 4,0 | F 24,4 | S 8,4 % |

L'acide de départ peut être préparé par le procédé décrit dans la demande européenne publiée N° 0041021.

*Exemple 26:*

*Poudre mouillable selon l'invention:*

On mélange:

| | |
|---|---|
| – Composé de l'exemple 1 (isomère Z) | 15 % |
| – Ekapersol S* | 10 % |
| – Brecolane N.V.A.** | 0,5% |
| – Zéosil 39*** | 34,5% |
| – Vercoryl S**** | 40 % |

* Produit de condensation du naphtalène sulfonate de sodium.
** Alcoyl naphtalène sulfonate de sodium.
*** Silice hydratée synthétique obtenue par précipitation.
**** Kaolin colloïdal.

*Exemple 27:*

*Concentré émulsifiable selon l'invention:*

On mélange:

| | |
|---|---|
| – Composé de l'exemple 1 (isomère Z) | 20 % |
| – Atlox 4851* | 6,4% |
| – Atlox 4855** | 3,2% |
| – Xylène | 70,4% |

* Mélange d'alkyl arylsulfonate et de triglycéride de polyoxyéthyléné; viscosité à 25° C: 300-700 cps.
** Mélange d'alkyl arylsulfonate et de triglycéride de polyoxyéthyléné; viscosité à 25° C: 1500-1900 cps.

*Exemple 28:*

*Concentré émulsifiable selon l'invention:*

On mélange:

| | |
|---|---|
| – Composé de l'exemple 1 (isomère Z) | 0,015 g |
| – Butoxyde de pipéronyle | 0,5 g |
| – Topanol A | 0,1 g |
| – Tween 80 | 3,5 g |
| – Xylène | 95,885 g |

*Exemple 29:*

*Concentré émulsifiable selon l'invention:*

On mélange:

– Composé de l'exemple 1 (isomère Z) 45 g

– Atlox 4851 6,4 g
– Atlox 4855 3,2 g
– Xylène 45,4 g

*Exemple 30:*

*Composition fumigène selon l'invention:*

– Composé de l'exemple 6 0,25 g
– Poudre de Tabu 25 g
– Poudre de feuille de cèdre 40 g
– Poudre de bois de pin 33,75 g
– Vert brillant 0,5 g
– p-nitrophénol 0,5 g

*Etude de l'activité des composés selon l'invention sur les parasites.*

*1°): Etude de l'effet létal sur mouches domestiques.*

Les insectes tests sont des mouches domestiques femelles âgées de 4/5 jours. On opère par application topique de 1 µl de solution acétonique du produit sur le thorax dorsal des insectes à l'aide du micro manipulateur d'Arnold. On utilise 50 individus par dose et par traitement. On effectue le contrôle de mortalité 24 h après traitement.

Le résultat obtenu exprimé en DL 50 ou dose (en nanogrammes) nécessaire pour tuer 50% des insectes est le suivant:

| Composé de l'exemple | DL 50 (ng par individu) |
|---|---|
| 1 (composé E) | 35,0 |
| 3 (composé Z) | 1,28 |
| 3 (composé E) | 58,3 |
| 2 (composé Z) | 0,98 |
| 4 (composé Z) | 5,1 |

*Conclusion:* Les produits testés présentent une activité létale sur mouche domestique.

*2°): Etude de l'effet sur larves de Spodoptera littoralis.*

Les essais sont effectués par application topique d'une solution acétonique du produit à tester à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24° C et 65% d'humidité relative. Après traitement, les individus sont placés sur un milieu nutritif artificiel (milieu de Poitout).

On effectue le contrôle des mortalités 48 h après traitement.

Le résultat expérimental obtenu est le suivant:

| Composé de l'exemple | DL 50 (ng par individu) |
|---|---|
| 1 (composé Z) | 40,0 |
| 1 (composé E) | 77,3 |
| 3 (composé Z) | 52,9 |
| 2 (composé Z) | 29,8 |

*Conclusion:* Les produits testés présentent une activité létale sur larves de Spodoptera littoralis.

*3°): Etude de l'activité de choc sur mouche domestique.*

Les insectes tests sont des mouches domestiques femelles âgées de 4/5 jours. On opère par pulvérisation directe en chambre de Kearns et March en utilisant comme solvant un mélange en volumes égaux d'acétone et d'isopar L (quantité de solution utilisée $2 \times 0,2\ cm^3$). On utilise environ 50 insectes par dose de traitement. On effectue les contrôles toutes les minutes jusqu'à 10 min, puis à 15 min et l'on détermine le KT 50 par les méthodes habituelles.

Les résultats obtenus sont les suivants:

| Composé de l'exemple | $KT_{50}$ en mn |
|---|---|
| 1 (composé Z) | 3,1 |
| 1 (composé E) | 4,9 |
| 3 (composé Z) | 5,4 |
| 3 (composé E) | 2,8 |
| 2 (composé Z) | 3,7 |

*Conclusion:* Les produits testés présentent une activité de choc sur mouche domestique.

*4°): Etude de l'activité par contact tarsal sur blatte germanique.*

Les insectes testés sont des mâles de blatte germanique (Blatella germanica). On opère par dépôt d'une solution acétonique de concentration déterminée sur le fond d'une boîte de Petri de 20 cm de diamètre. Après séchage, on laisse séjourner 20 blattes mâles par concentration durant une heure, puis on transfère les insectes sur milieu sain et on contrôle leur mortalité à 24 h, 48 h, 3 et 5 jours.

Les résultats expérimentaux, exprimés en concentration létale 50 (CL 50) sont résumés dans le tableau suivant:

| Composé de l'exemple | CL 50 en $mg/m^2$ |
|---|---|
| 1 (composé Z) | 0,25 |
| 3 (composé Z) | 0,21 |
| 2 (composé Z) | 0,18 |
| 4 (composé Z) | 0,17 |

*Conclusion:* Les composés testés sont doués d'une activité létale vis-à-vis de Blatella germanica.

*5°): Etude de l'activité sur la bruche du haricot (Acanthocelida sobtectus).*

L'essai est effectué par application topique de 1 µl de solution acétonique du produit à tester sur le thorax de l'insecte.

On détermine les DL 50 exprimés en nanogrammes par insecte. Les résultats expérimentaux sont résumés dans le tableau suivant:

| Composé de l'exemple | DL 50 en ng/insecte |
|---|---|
| 1 (composé Z) | 7,5 |
| 3 (composé Z) | 13,0 |
| 2 (composé Z) | 17,7 |
| 4 (composé Z) | 32,6 |

*Conclusion:* Les composés testés sont doués d'une activité insecticide vis-à-vis de la bruche du haricot.

*6°): Activité sur Tetranychus urticae.*

Essai adulticide.

On utilise des plants de haricot comportant deux feuilles cotylédonaires. Ces plants sont traités au pistolet Fisher avec une solution acétonique du produit à tester. Après séchage, 25 femelles de l'acarien Tetranychus urticae sont disposées sur chaque feuille, soit 50 individus par dose expérimentée par plant. Les contrôles d'efficacité sont effectués après 1, 24, 48 et 72 heures de contact.

Dans ce test, les produits des exemples 1 à 8 sont doués d'une activité acaricide sur Tetranychus urticae.

*7°): Activité sur Panagrellus silusiae.*

Dans un récipient d'environ 50 $cm^3$, on introduit des nématodes (environ 2000), en suspension dans 0,5 $cm^3$ d'eau. On ajoute à cette suspension 10 $cm^3$ de solution aqueuse du produit à tester, aux concentrations de 1 ou 0,1 g/l. On effectue 3 répétitions par concentration.

Au bout de 24 heures, on homogénéise le milieu aqueux, on effectue un prélèvement de 1 ml et dénombre, sur lame de Peter, les nématodes vivants et morts. On compare les résultats à ceux obtenus avec un témoin non traité.

Dans ce test, les produits des exemples 1 à 8 sont doués d'une activité numaticide sur Panagrellus silusiae.

## Revendications

1. Sous toutes leurs formes stéréo-isomères ou sous forme de mélanges, les composés de formule (I):

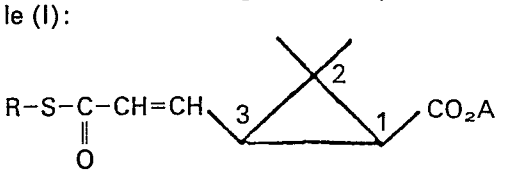

(I)

dans laquelle la copule cyclopropanique a la structure 1R cis et la double liaison a la géométrie Z et dans laquelle A représente

– soit un radical alcoyle renfermant de 1 à 18 atomes de carbone,

– soit un radical benzyle éventuellement substitué sur le cycle aromatique par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

– soit un groupement

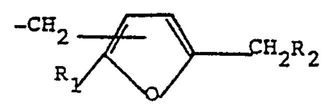

dans lequel le substituant $R_1$ représente un atome d'hydrogène ou un radical méthyle et le substituant $R_2$ un aryle monocyclique ou un groupement $-CH_2-C{\equiv}CH$ et notamment un groupement 5-benzyl 3-furyl méthyle,

– soit un groupement

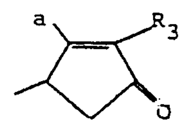

dans lequel a représente un atome d'hydrogène ou un radical méthyle et $R_3$ représente un radical organique aliphatique comportant de 2 à 6 atomes de carbone et une ou plusieurs insaturations carbone-carbone et notamment l'un des radicaux $-CH_2-CH{=}CH_2$, $-CH_2-CH{=}CH-CH_3$, $-CH_2-CH{=}CH-CH{=}CH_2$ ou $-CH_2-CH{=}CH-CH_2-CH_3$,

– soit un groupement

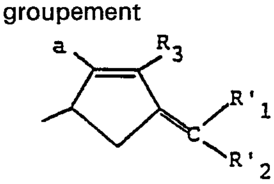

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

– soit un groupement

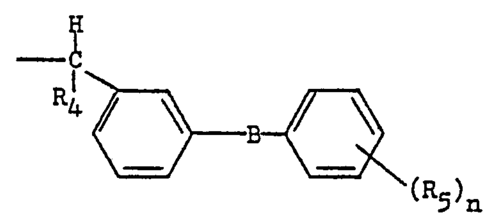

dans lequel B représente un atome d'oxygène ou de soufre ou un groupement $-\overset{O}{\overset{\|}{C}}-$ ou $-CH_2-$ et $R_4$ représente un atome d'hydrogène, un radical $-C{\equiv}N$, un radical méthyle, un radical $-CONH_2$, un radical $-CSNH_2$ ou un radical $-C{\equiv}CH$, $R_5$ représente un atome d'halogène ou un radical méthyle et n représente un nombre égal à 0, 1 ou 2, et notamment le groupement 3-phénoxy benzyle, α-cyano 3-phénoxy benzyle, α-éthynyl 3-phénoxy benzyle, 3-benzoyl benzyle, 1-(3-phénoxy phényl) éthyle ou α-thioamido 3-phénoxy benzyle,

– soit un groupement

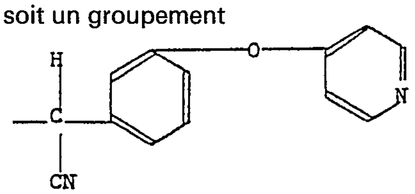

– soit un groupement

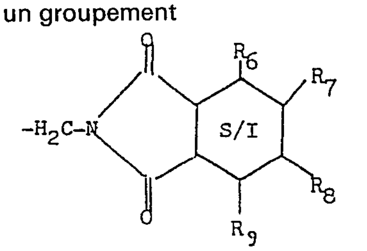

dans lequel les substituants $R_6$, $R_7$, $R_8$, $R_9$ représentent un atome d'hydrogène, un atome de chlore ou un radical méthyle et dans lequel S/I symbolise un cycle aromatique ou un cycle analogue dihydro ou tétrahydro,

– soit un groupement

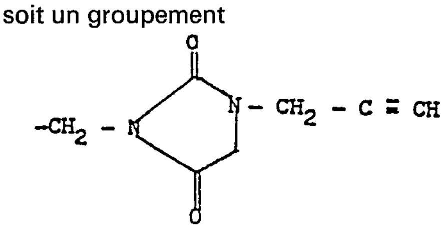

– soit un groupement

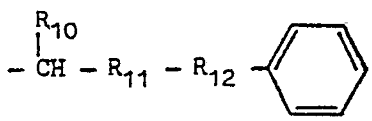

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec $-\underset{R_{10}}{\underset{|}{CH}}-$ peut se trouver à l'une quelconque des positions disponibles, $R_{12}$ étant lié à $R_{11}$ par l'atome de carbone compris entre l'atome de soufre et un atome d'azote,

– soit un groupement

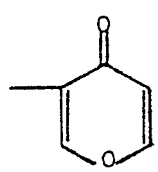

– soit un groupement

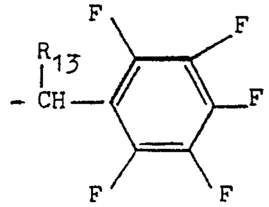

dans lequel $R_{13}$ représente un atome d'hydrogène ou un radical CN,

– soit un groupement

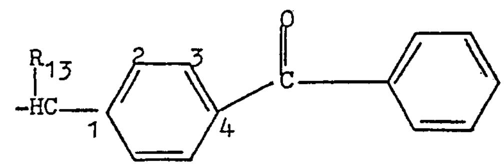

dans lequel $R_{13}$ est défini comme ci-dessus, et le radical benzoyle est en position 4,

– soit un groupement

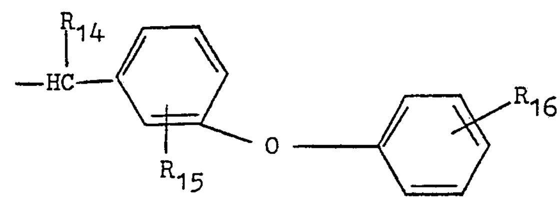

dans lequel $R_{14}$ représente un atome d'hydrogène, un radical méthyle, éthynyle ou cyano, $R_{15}$ représente un atome de fluor, de chlore ou de brome et $R_{16}$ représente un atome d'hydrogène, de fluor, de chlore ou de brome,

– soit un groupement

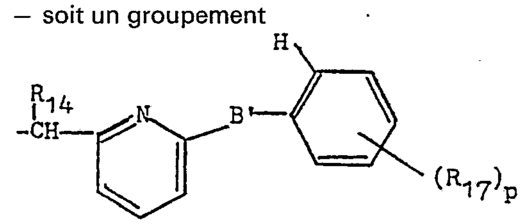

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre, R représente un radical alcoyle renfermant de 1 à 18 atomes de carbone linéaire, ramifié, saturé ou insaturé, éventuellement substitué par un ou plusieurs groupements fonctionnels identiques ou différents, choisis dans le groupe constitué par les atomes d'halogène, les groupements OH ou SH, les groupements OR' ou SR' dans lesquels R' représente un radical alcoyle renfermant de 1 à 8 atomes de carbone, les groupements $NO_2$, les groupements:

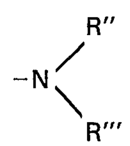

dans lesquels R'' et R''', identiques ou différents, représentent un atome d'hydrogène, ou un radical alcoyle renfermant de 1 à 8 atomes de carbone, ou R' représente un groupement: $-C{\equiv}N$, $SO_3H$ ou $PO_4H_2$ ou un groupement $COalc_1$, $SO_2alc_2$ ou $SO_3alc_3$ dans lesquels $alc_1$, $alc_2$ et $alc_3$ représentent des radicaux alcoyle renfermant de 1 à 18 atomes de carbone, ou bien R représente un radical alcoyl cyclique, saturé ou insaturé, comportant de 3 à 7 atomes de carbone, éventuellement substitué par un ou plusieurs groupements fonctionnels, identiques ou différents, choisis dans le groupe constitué par les atomes d'halogène, les radicaux alcoyle comportant de 1 à 6 atomes de carbone, les radicaux alcoyloxyle comportant de 1 à 6 atomes de carbone, et les groupements $NO_2$, ou bien R représente un groupement aryle renfermant de 6 à 14 atomes de carbone, éventuellement substitué par un ou plusieurs groupements fonctionnels identiques ou différents, choisis dans le groupe constitué par les groupements OH, Oalc ou alc renfermant de 1 à 8 atomes de carbone, par un groupement $CF_3$, $OCF_3$ ou $SCF_3$ ou par un atome de chlore, de brome ou de fluor, ou bien R représente un radical hétérocyclique.

2. Les composés de formule (I), tels que définis à la revendication 1, pour lesquels A représente un groupement α-cyano 3-phénoxybenzyle sous forme S, R ou RS.

3. Les composés de formule (I), tels que définis à la revendication 1, pour lesquels A représente un groupement /3-(propyn-2-yl) 2,5-dioxo imidazolidinyl/méthyle.

4. Les composés de formule (I), tels que définis à l'une quelconque des revendications 1 à 3, pour lesquels R représente un radical alcoyle linéaire ou ramifié renfermant de 1 à 18 atomes de carbone.

5. Les composés de formule (I), tels que définis à la revendication 4, caractérisés en ce que R représente un radical méthyle, éthyle, isopropyle ou terbutyle.

6. Procédé de préparation des composés de formule (I), tels que définis à l'une quelconque des revendications 1 à 5, caractérisé en ce que l'on fait réagir un acide de formule (II):

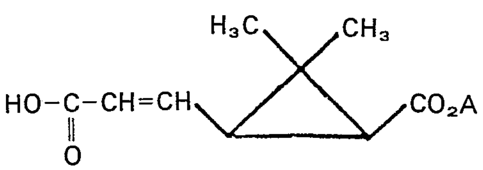 (II)

formule dans laquelle la copule cyclopropanique a la structure 1R cis et la double liaison a la géométrie Z et dans laquelle A conserve les significations de la revendication 1, au sein d'un solvant organique, en présence de dicyclohexyl carbodiimide, avec un mercaptan de formule (III):

$$HS-R \qquad (III)$$

formule dans laquelle R conserve les significations de la revendication 1.

7. Application des composés de formule (I), tels que définis à l'une quelconque des revendications 1 à 5, à la lutte contre les parasites des végétaux, les parasites des locaux et les parasites des animaux à sang chaud.

8. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 5.

9. Les compositions insecticides renfermant comme principe actif au moins l'un des produits définis à l'une quelconque des revendications 1 à 5.

10. Les compositions destinées à l'alimentation animale renfermant comme principe actif au moins l'un des produits définis à l'une quelconque des revendications 1 à 5.

11. A titre de médicaments, les composés définis à l'une quelconque des revendications 1 à 5.

12. Les compositions pharmaceutiques renfermant comme principe actif l'un au moins des médicaments définis à la revendication 11.

13. Associations douées d'activité insecticide, acaricide ou nématicide, caractérisées en ce qu'elles contiennent comme matière active, d'une part un au moins des composés de formule générale (I), tels que définis à la revendication 1, et d'autre part un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels «halo» représente un atome de fluor, de chlore ou de brome, étant entendu que les composés (I) peuvent exister sous toutes leurs formes stéréoisomères possibles, de même que les copules acides et alcools des esters pyréthrinoïdes ci-dessus.

**Patentansprüche**

1. Die Verbindungen der Formel I in allen ihren stereoisomeren Formen oder in der Form von Gemischen:

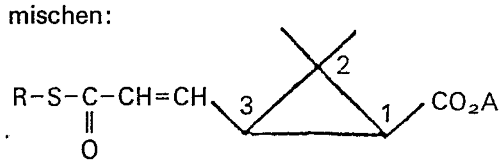 (I)

worin die Cyclopropankombination die Struktur 1R-cis und die Doppelbindung die Geometrie Z hat und worin A darstellt:

– entweder einen Alkylrest mit 1 bis 18 Kohlenstoffatomen,

– oder einen Benzylrest, gegebenenfalls substituiert am aromatischen Zyklus durch einen oder mehrere Reste, ausgewählt aus der Gruppe von den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,

– oder eine Gruppe

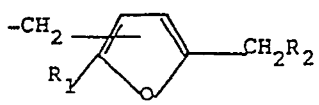

worin der Substituent $R_1$ ein Wasserstoffatom oder einen Methylrest bedeutet und der Substituent $R_2$ ein monocyclisches Aryl oder eine Gruppe $-CH_2-C\equiv CH$ und insbesondere eine 5-Benzyl-3-furylmethyl-Gruppe bedeutet,

– oder eine Gruppe

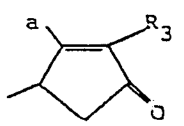

worin a ein Wasserstoffatom oder einen Methylrest bedeutet und $R_3$ einen organischen aliphatischen Rest mit 2 bis 6 Kohlenstoffatomen und einer oder mehreren Kohlenstoff-Kohlenstoff Unsättigungen, und insbesondere einen der Reste $-CH_2-CH=CH_2$, $-CH_2-CH=CH-CH_3$, $-CH_2-CH=CH-CH=CH_2$ oder $-CH_2-CH=CH-CH_2-CH_3$ bedeutet,

– oder eine Gruppe

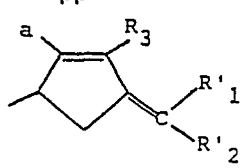

worin a ein Wasserstoffatom oder einen Methylrest bedeutet, $R_3$ die gleiche Bedeutung wie vorstehend aufweist, $R'_1$ und $R'_2$, die gleich oder verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkyloxycarbonylgruppe mit 2 bis 5 Kohlenstoffatomen oder eine Cyanogruppe darstellen,

– oder eine Gruppe

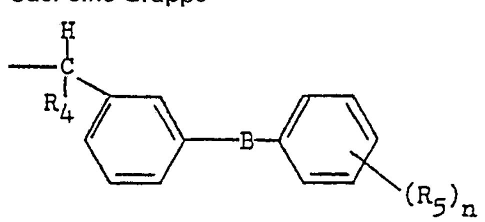

worin B ein Sauerstoff- oder Schwefelatom oder eine Gruppe $-\overset{\overset{O}{\|}}{C}-$ oder $-CH_2-$ ist und $R_4$ ein Wasserstoffatom, einen Rest $-C\equiv N$, einen Methylrest, einen Rest $-CONH_2$, einen Rest $-CSNH_2$ oder einen Rest $-C\equiv CH$ bedeutet, $R_5$ ein Halogenatom oder einen Methylrest darstellt und n eine der Zahlen 0, 1 oder 2 bedeutet, und insbesondere die 3-Phenoxybenzylgruppe, α-Cyano-3-phenoxybenzyl-Gruppe, α-Ethinyl-3-phenoxybenzyl-Gruppe, 3-Benzoylbenzyl-Gruppe, 1-(3-Phenoxyphenyl)-ethyl-Gruppe oder α-Thioamido-3-phenoxybenzyl-Gruppe,

– oder eine Gruppe

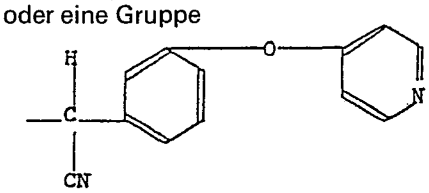

– oder eine Gruppe

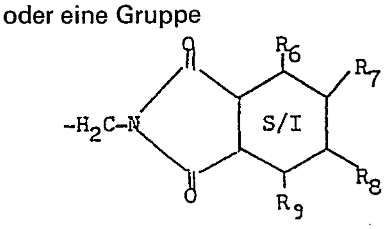

worin die Substituenten $R_6$, $R_7$, $R_8$, $R_9$ ein Wasserstoffatom, ein Chloratom oder einen Methylrest bedeuten und worin S/I einen aromatischen Ring oder einen analogen Dihydro- oder Tetrahydroring bedeutet,

– oder eine Gruppe

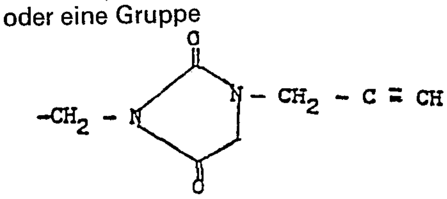

– oder eine Gruppe

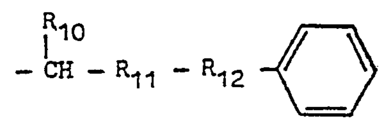

worin $R_{10}$ ein Wasserstoffatom oder einen Rest CN bedeutet, $R_{12}$ einen Rest $-CH_2-$ oder ein Sauerstoffatom bedeutet, $R_{11}$ einen Thiazolyl- oder Thiadiazolylrest bedeutet, dessen Bindung mit $-\underset{\underset{R_{10}}{|}}{CH}-$ sich an einer beliebigen verfügbaren Stelle befinden kann, $R_{12}$ an $R_{11}$ über das Kohlenstoffatom gebunden ist, das sich zwischen dem Schwefelatom und dem Stickstoffatom befindet,

– oder eine Gruppe

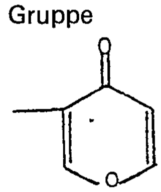

– oder eine Gruppe

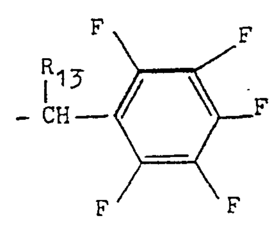

worin $R_{13}$ ein Wasserstoffatom oder einen Rest CN bedeutet,

– oder eine Gruppe

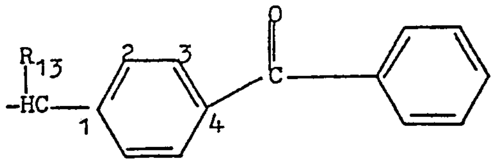

worin $R_{13}$ wie vorstehend definiert ist und der Benzoylrest in der 4-Stellung ist,

– oder eine Gruppe

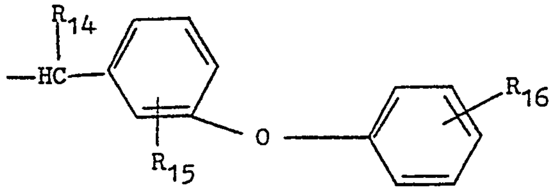

worin $R_{14}$ ein Wasserstoffatom, einen Methyl-, Ethinyl- oder Cyanorest bedeutet, $R_{15}$ ein Fluor-, Chlor- oder Bromatom bedeutet und $R_{16}$ ein Wasserstoff-, Fluor-, Chlor- oder Bromatom darstellt,

– oder eine Gruppe

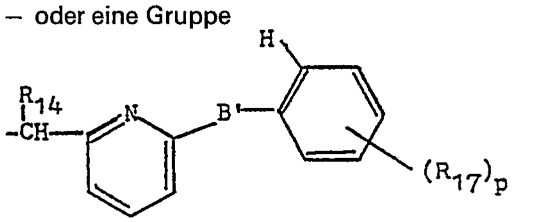

worin $R_{14}$ wie vorstehend definiert ist, wobei jedes von $R_{17}$ unabhängig eine Alkylgruppe mit 1 bis 4 Kohlenstoffatomen, Alkoxygruppe mit 1 bis 4 Kohlenstoffatomen, Alkylthiogruppe mit 1 bis 4 Kohlenstoffatomen, Alkylsulfonylgruppe mit 1 bis 4 Kohlenstoffatomen, Trifluormethylgruppe, 3,4-Methylendioxygruppe, Chlor-, Fluor- oder Bromgruppe darstellt, p eine der Zahlen 0, 1 oder 2 bedeutet und B' ein Sauerstoffatom oder ein Schwefelatom darstellt, R einen Alkylrest mit 1 bis 18 Kohlenstoffatomen, der linear, verzweigt, gesättigt oder ungesättigt sein kann, gegebenenfalls substituiert durch eine oder mehrere funktionelle gleiche oder verschiedene Gruppen, ausgewählt aus der Gruppe von den Halogenatomen, den OH- oder SH-Gruppen, den OR'- oder SR'-Gruppen, worin R' einen Alkylrest mit 1 bis 8 Kohlenstoffatomen bedeutet, den Gruppen $NO_2$, den Gruppen

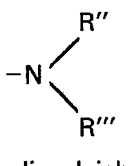

worin R'' und R''', die gleich oder verschieden sind, ein Wasserstoffatom, einen Alkylrest mit 1 bis 8 Kohlenstoffatomen bedeutet, oder R' eine Gruppe $-C{\equiv}N$, $SO_3H$ oder $PO_4H_2$ oder eine Gruppe $COAlk_1$, $SO_2Alk_2$ oder $SO_3Alk_3$ bedeutet, worin $Alk_1$, $Alk_2$ und $Alk_3$ Alkylreste mit 1 bis 18 Kohlenstoffatomen bedeuten, darstellt, oder R einen zyklischen gesättigten oder ungesättigten Alkylrest mit 3 bis 7 Kohlenstoffatomen, gegebenenfalls substituiert durch eine oder mehrere funktionelle Gruppen, die gleich oder verschieden sind, ausgewählt aus der Gruppe von den Halogenatomen, Alkylresten mit 1 bis 6 Kohlenstoffatomen, den Alkyloxyresten mit 1 bis 6 Kohlenstoffatomen, den Gruppen $NO_2$, bedeutet, oder R eine Arylgruppe mit 6 bis 14 Kohlenstoffatomen, gegebenenfalls substituiert durch eine oder mehrere gleiche oder verschiedene funktionelle Gruppen, ausgewählt aus der Gruppe von den Gruppen OH, OAlk, worin Alk 1 bis 8 Kohlenstoffatome hat, einer Gruppe $CF_3$, $OCF_3$ oder $SCF_3$ oder einem Chlor-, Brom- oder Fluoratom bedeutet, oder R einen heterocyclischen Rest darstellt.

2. Verbindungen der Formel I gemäss Anspruch 1, worin A eine α-Cyano-3-phenoxybenzyl-Gruppe in der S-, R- oder RS-Form darstellt.

3. Verbindungen der Formel I, wie in Anspruch 1 definiert, worin A eine [3-(Propin-2-yl)-2,5-dioxoimidazolidinyl]-methylgruppe bedeutet.

4. Verbindungen der Formel I, wie in einem der Ansprüche 1 bis 3 definiert, worin R einen linearen oder verzweigten Alkylrest mit 1 bis 18 Kohlenstoffatomen bedeutet.

5. Verbindungen der Formel I, wie in Anspruch 4 definiert, dadurch gekennzeichnet, dass R einen Methyl-, Ethyl-, Isopropyl- oder tert-Butylrest bedeutet.

6. Verfahren zur Herstellung der Verbindungen der Formel I, wie in einem der Ansprüche 1 bis 5 definiert, dadurch gekennzeichnet, dass man eine Säure der Formel II

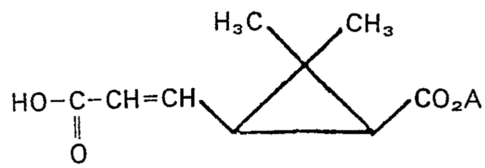
(II)

worin die Cyclopropankombination die Struktur 1R-cis und die Doppelbindung die Geometrie Z aufweist und worin A die Bedeutungen des Anspruchs 1 beibehält, in einem organischen Lösungsmittel in Anwesenheit von Dicyclohexylcarbodiimid mit einem Mercaptan der Formel III

$$HS-R \quad (III)$$

wobei in der Formel R die im Anspruch 1 angegebenen Bedeutungen beibehält, umsetzt.

7. Verwendung der Verbindungen der Formel I, wie in einem der Ansprüche 1 bis 5 definiert, zur Bekämpfung von Pflanzenparasiten, Raumparasiten und Parasiten von Warmblütertieren.

8. Zusammensetzungen, bestimmt zur Bekämpfung von Pflanzenparasiten, Raumparasiten und Parasiten von Warmblütertieren, dadurch gekennzeichnet, dass sie als aktives Prinzip mindestens eine der Verbindungen, definiert gemäss einem der Ansprüche 1 bis 5, enthalten.

9. Insektizide Zusammensetzungen, enthaltend als aktives Prinzip mindestens eine der Verbindungen, definiert in einem der Ansprüche 1 bis 5.

10. Zusammensetzungen, bestimmt zur tierischen Ernährung, enthaltend als aktives Prinzip mindestens eine der Verbindungen, definiert in einem der Ansprüche 1 bis 5.

11. Als Arzneimittel die Verbindungen definiert in einem der Ansprüche 1 bis 5.

12. Pharmazeutische Zusammensetzungen, enthaltend als wirksames Prinzip mindestens eines der Arzneimittel, definiert im Anspruch 11.

13. Zusammensetzungen mit insektizider, akarizider oder nematizider Wirkung, dadurch gekennzeichnet, dass sie als aktives Material enthalten: einerseits mindestens eine der Verbindungen der allgemeinen Formel I, wie in Anspruch 1 definiert, und andererseits mindestens einen der Pyrethrinoid-Ester, ausgewählt aus der Gruppe von Allethrolon-Estern von 3,4,5,6-Tetrahydrophthalimidomethylalkohol, 5-Benzyl-3-furylmethylalkohol, 3-Phenoxybenzylalkohol und den α-Cyano-3-phenoxybenzylalkoholen der Chrysanthemumsäuren, den Estern von 5-Benzyl-3-furylmethylalkohol der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole mit 2,2-Dimethyl-3-(2,2-dibromvinyl)-cyclopropan-1-carbonsäuren, den Estern von 3-Phenoxybenzylalkohol mit den 2-p-Chlorphenyl-2-isopropylessigsäuren, den Allethrolonestern von 3,4,5,6-Tetrahydrophthalimidomethylalkohol, 5-Benzyl-3-furylmethylalkohol, 3-Phenoxybenzylalkohol und den α-Cyano-3-phenoxybenzylalkoholen, der 2,2-Dimethyl-3-(1,2,2,2-tetrahalogenethyl)-cyclopropan-1-carbonsäuren, worin «Halogen» ein Fluor-, Chlor- oder Bromatom bedeutet, wobei selbstverständlich die Verbindungen I in ihren sämtlichen möglichen stereoisomeren Formen vorliegen können, wie auch die Verbindungen von Säuren und Alkoholen der vorstehenden Pyrethrinoid-Ester.

## Claims

1. In all their stereoisomeric forms or in the form of mixtures, compounds with the formula (I):

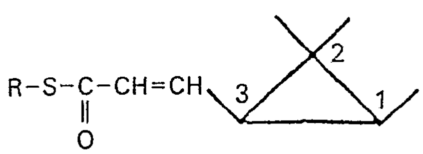 (I)

in which the cyclopropane copula has the 1R cis structure and the double bond has the geometry Z and in which A represents

– either an alkyl radical containing from 1 to 18 carbon atoms,

– or a benzyl radical possibly substituted on the aromatic ring by one or more radicals chosen from the group composed of the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylene dioxy radical and the halogen atoms,

– or a group

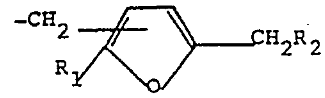

in which the substituent $R_1$ represents a hydrogen atom or a methyl radical and the substituent $R_2$ a monocyclic aryl radical or a group $-CH_2-C\equiv CH$ and in particular a methyl 5-benzyl 3-furyl group,

– or a group

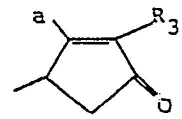

in which a represents a hydrogen atom or a methyl radical and $R_3$ represents an organic aliphatic radical containing from 2 to 6 carbon atoms and one or more carbon-carbon unsaturations and in particular one of the radicals $-CH_2-CH=CH_2$, $-CH_2-CH=CH-CH_3$, $-CH_2-CH=CH-CH=CH_2$ or $-CH_2-CH=CH-CH_2-CH_3$,

– or a group

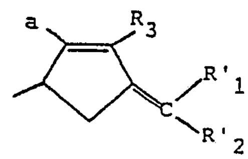

in which a represents a hydrogen atom or a methyl radical, $R_3$ retains the same significance as previously, $R'_1$ and $R'_2$, identical or different, each represent a hydrogen atom, a halogen atom, an alkyl radical containing from 1 to 6 carbon atoms, an aryl radical including from 6 to 10 carbon atoms, an alkyloxycarbonyl group including from 2 to 5 carbon atoms, or a cyano group,

– or a group

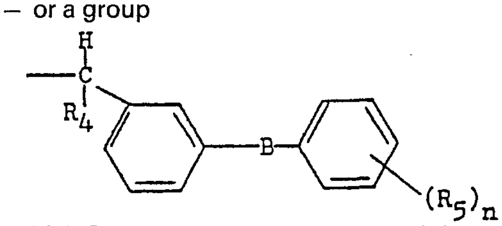

in which B represents an oxygen or sulphur atom or a $-\overset{O}{\overset{\|}{C}}-$ or $-Ch_2$ and $R_4$ represents a hydrogen atom, a $-C\equiv N$ radical, a methyl radical, a $-CONH_2$ radical, a $-CSNH_2$ radical or a $-C\equiv CH$ radical, $R_5$ represents a halogen atom or a methyl radical and n represents a number 0, 1 or 2, and in particular the group 3-phenoxy benzyl, α-cyano-3-phenoxybenzyl, α-ethynyl-3-phenoxybenzyl, 3-benzoyl benzyl, 1-(3-phenoxyphenyl)ethyl or α-thioamido-3-phenoxybenzyl,

– or a group

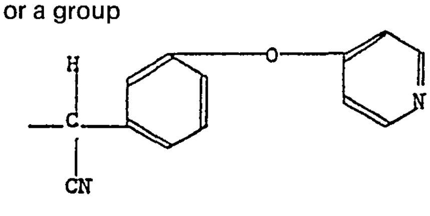

— or a group

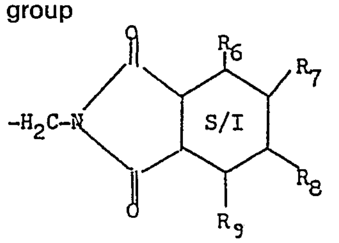

in which the substituents $R_6$, $R_7$, $R_8$ and $R_9$ each represent a hydrogen atom, a chlorine atom or a methyl radical and in which S/I symbolises an aromatic ring or a similar dihydro or tetrahydro ring,

— or a group

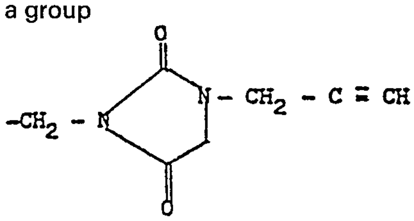

— or a group

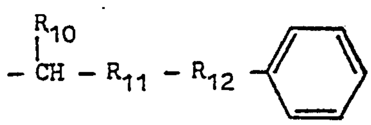

in which $R_{10}$ represents a hydrogen atom or a CN radical, $R_{12}$ represents a $-CH_2-$ radical or an oxygen atom, $R_{11}$ represents a thiazolyl or thiadiazolyl radical, of which the bond with $-\underset{R_{10}}{\underset{|}{CH}}-$ can be found at any one of the available positions, $R_{12}$ being attached to $R_{11}$ by the carbon atom included between the sulphur atom and a nitrogen atom,

— or a group

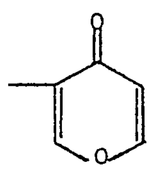

— or a group

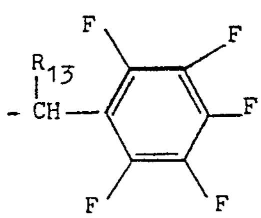

in which $R_{13}$ represents a hydrogen atom or a CN radical,

— or a group

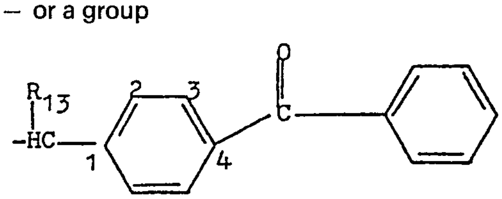

in which $R_{13}$ is defined as above, and the benzoyl radical is at position 4,

— or a group

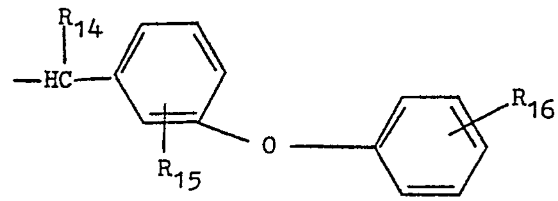

in which $R_{14}$ represents a hydrogen atom, a methyl, ethynyl or cyano radical, $R_{15}$ represents a fluorine, chlorine or bromine atom and $R_{16}$ represents a hydrogen, fluorine, chlorine or bromine atom,

— or a group

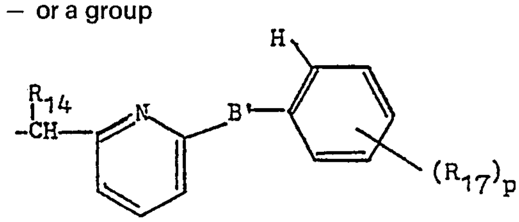

in which $R_{14}$ is defined as above, each of the $R_{17}$'s represents independently an alkyl group containing from 1 to 4 carbon atoms, an alkoxy group containing from 1 to 4 carbon atoms, an alkylthio group containing from 1 to 4 carbon atoms, an alkylsulphonyl group containing from 1 to 4 carbon atoms, a trifluoromethyl, 3,4-methylenedioxy, chloro, fluoro or bromo group, p represents a number 0, 1 or 2, and B' represents an oxygen atom or a sulphur atom, R represents an alkyl radical containing from 1 to 18 carbon atoms, linear or branched, saturated or unsaturated, possibly substituted by one or more functional groups, identical or different, chosen from the group constituted by the halogen atoms, the OH or SH groups, the OR' or SR' groups in which R' represents an alkyl radical containing from 1 to 8 carbon atoms, the $NO_2$ groups, the groups:

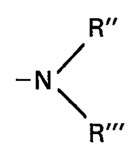

in which R'' and R''', identical or different, each represent a hydrogen atom, or an alkyl radical containing from 1 to 8 carbon atoms, or R' represents a group: $-C{\equiv}N$, $SO_3H$ or $PO_4H_2$ or a group $COalk_1$, $SO_2alk_2$ or $SO_3alk_3$ in which $alk_1$, $alk_2$ and $alk_3$ represent alkyl radicals containing from 1 to 18 carbon atoms, or R represents a cyclic alkyl radical, saturated or unsaturated, containing from 3 to 7 carbon atoms, possibly substituted by one or more functional groups, identical or different, chosen from among the group composed of the halogen atoms, the alkyl radicals including from 1 to 6 carbon atoms, the alkyloxyl radicals including from 1 to 6 carbon atoms, and the $NO_2$ group, or R represents an aryl group containing from 6 to 14 carbon atoms, possibly substituted by one or more functional groups, identical or different, chosen from the group composed of the OH, Oalk or alk groups, containing from 1 to 8 carbon atoms

by a $CF_3$, $OCF_3$ or $SCF_3$ group or by a chlorine, bromine or fluorine atom, or R represents a heterocyclic radical.

2. Compounds with the formula (I), as defined in claim 1, for which A represents an α-cyano 3-phenoxybenzyl group in the form S, R or RS.

3. Compounds with the formula (I), as defined in claim 1, for which A represents a [3-(propyn-2-yl)-2,5-dioxo imidazolidinyl]methyl group.

4. Compounds with the formula (I), as defined in any one of the claims 1 to 3, for which R represents an alkyl radical, linear or branched containing from 1 to 18 carbon atoms.

5. Compounds with the formula (I), as defined in claim 4, characterized in that R represents a methyl, ethyl, isopropyl or tertbutyl radical.

6. Preparation process of compounds with the formula (I), as defined in any one of the claims 1 to 5, characterized in that an acid with the formula (II):

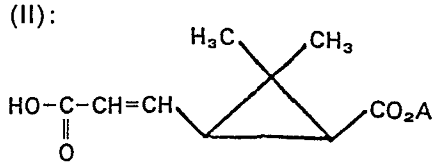 (II)

in which formula the cyclopropane copula has the structure 1R cis and the double bond has the geometry Z and in which A retains the significances of claim 1, in an organic solvent, in the presence of dicyclohexyl carbodiimide, is made to react with a mercaptan with the formula (III):

HS−R (III)

in which formula R retains the significances of claim 1.

7. Use of compounds with the formula (I), as defined in any one of the claims 1 to 5, in combating parasites of vegetation, parasites of premises and parasites of warm-blooded animals.

8. Compositions intended for combating parasites of vegetation, parasites of premises and parasites of warm-blooded animals, characterized in that they contain as active principle at least one of the products defined in any one of the claims 1 to 5.

9. Insecticide compositions containing as active principle at least one of the products defined in any one of the claims 1 to 5.

10. Compositions intended for animal feeding containing as active principle at least one of the products defined in any one of the claims 1 to 5.

11. As medicaments, compounds defined in any one of the claims 1 to 5.

12. Pharmaceutical compositions containing as active principle, at least one of the medicaments defined in claim 11.

13. Combinations endowed with insecticide, acaricide or nematocide activity, characterized in that they contain as active material, on the one hand at least one of the compounds with the general formula (I), as defined in claim 1, and, on the other hand, one at least of the pyrethrinoid esters chosen from the group constituted by the esters of allethrolones, of 3,4,5,6-tetrahydrophthalimidomethyl alcohol, of 5-benzyl 3-furylmethyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-3-tetrahydrothiophenylidenemethyl) cyclopropane-1-carboxylic acids, by the esters of 3-phenoxybenzyl alcohol and α-cyano-3-phenoxybenzyl alcohols with 2,2-dimethyl-3-(2,2-dichlorovinyl) cyclopropane-1-carboxylic acids, by the esters of α-cyano-3-phenoxybenzyl alcohols with 2,2-dimethyl-3-(2-dibromovinyl)cyclopropane-1-carboxylic acids, by the esters of 3-phenoxybenzyl alcohol with 2-parachlorophenyl-2-isopropylacetic acids, by the esters of allethrolones, of 3,4,5,6-tetrahydrophthalimidomethyl alcohol, of 5-benzyl-3-furylmethyl alcohol, of 3-phenoxybenzyl alcohol and α-cyano-3-phenoxybenzyl alcohols with 2,2-dimethyl-3-(1,2,2,2-tetrahaloethyl) cyclopropane-1-carboxylic acids, in which «halo» represents a fluorine, chlorine or bromine atom, it being understood that the compounds (I) can exist in all their possible stereo-isomeric forms, as can the acid and alcohol copulas of the above pyrethrinoid esters.